# EUROPÄISCHE PATENTSCHRIFT

(11) **EP 1 979 337 B1**
(45) Veröffentlichungstag und Bekanntmachung des Hinweises auf die Patenterteilung: **25.05.2011**
(21) Anmeldenummer: 07702622.7
(22) Anmeldetag: 09.01.2007
(51) Int. Cl.: C07D 311/22, A61K 8/34

(54) **CHROMEN-4-ON-DERIVATE ALS SELBSTBRÄUNUNGSSUBSTANZ**
CHROMEN-4-ONE DERIVATIVES AS A SELF-TANNING SUBSTANCE
DÉRIVÉS DE CHROMÈNE-4-ONE COMME SUBSTANCE AUTOBRONZANTE

(30) Priorität: 31.01.2006 DE 102006004327
(43) Veröffentlichungstag der Anmeldung: 15.10.2008
(73) Patentinhaber: Merck Patent GmbH, 64293 Darmstadt (DE)
(72) Erfinder: CAROLA, Christophe, 69120 Heidelberg (DE); TOULLEC, Anne, 92160 Antony (FR); BUCHHOLZ, Herwig, 60599 Frankfurt (DE)
(86) Internationale Anmeldenummer: PCT/EP2007/000106
(87) Internationale Veröffentlichungsnummer: WO 2007/087956

(56) Entgegenhaltungen:
- EP-A- 1 508 327
- WO-A-2005/097772

## Beschreibung

Gegenstand der vorliegenden Erfindung ist die Verwendung von Chromen-4-on-Derivaten als Selbstbräunungswirkstoffe sowie neue Chromen-4-on-Derivate und ihre Herstellung. Ferner betrifft die Erfindung Zubereitungen mit einem wirksamen Gehalt an solchen Chromen-4-on-Derivaten.

Der Trend weg von der vornehmen Blässe hin zur "gesunden, sportlich braunen Haut" ist seit Jahren ungebrochen. Um diese zu erzielen, setzen die Menschen ihre Haut der Sonnenstrahlung aus, da diese eine Pigmentierung durch Melaninbildung hervorruft. Die UV-Strahlung des Sonnenlichtes hat jedoch auch eine schädigende Wirkung auf die Haut. Neben der akuten Schädigung (Sonnenbrand) treten Langzeitschäden bei übermäßiger Bestrahlung mit Licht aus dem UVB-Bereich (Wellenlänge 280-320 nm) auf, wie beispielsweise ein erhöhtes Risiko an Hautkrebs zu erkranken. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Den natürlichen Schutz vor den negativen Folgen der Sonnenstrahlung bietet die Bräunung (Pigmentierung) der Haut. Die Epidermis enthält in ihrer untersten Schicht, der Basalschicht, neben den Basalzellen einzelne pigmentbildende Zellen, die Melanocyten. Durch UV-Licht wird in diesen Zellen die Produktion von Melanin angeregt, das in die Kerantinocyten (Hornzellen) transportiert und dort als braune Hautfarbe sichtbar wird. Diese von der Aminosäure Tyrosin ausgehende Pigmentbildung wird überwiegend durch UVB-Strahlung initiiert und als "indirekte Pigmentierung" bezeichnet. Ihre Entwicklung läuft über mehrere Tage; die so erhaltene Sonnenbräune besteht über einige Wochen.

Bei der "Direkt-Pigmentierung", die mit der Sonnenbestrahlung einsetzt, werden vorwiegend farblose Melanin-Vorstufen durch UVA-Strahlung zu dunkel gefärbtem Melanin oxidiert. Da diese Oxidierung reversibel ist, führt sie zu einer nur kurz anhaltenden Hautbräunung.

Eine künstliche Bräunung der Haut lässt sich äußerlich mit Hilfe von Schminke und oral durch Einnahme von Carotinoiden erzeugen. Weitaus beliebter jedoch ist die künstliche Bräunung der Haut, welche sich durch Auftragen von sogenannten Selbstbräunern erzielen lässt. Diese Verbindungen weisen als chemisches Strukturmerkmal Keto- bzw. Aldehydgruppen in Nachbarschaft zu Alkoholfunktionen auf. Diese Ketole bzw. Aldole gehören überwiegend zur Substanzklasse der Zucker. Eine besonders häufig eingesetzte Selbstbräunungssubstanz ist 1,3-Dihydroxyaceton (DHA) und Erythrulose (1,3,4-Trihydroxy-2-butanon). Diese Verbindungen können mit den Proteinen und Aminosäuren der Hornschicht der Haut im Sinne einer Maillard-Reaktion umgesetzt werden, wobei über einen noch nicht vollständig aufgeklärten Reaktionsweg Polymerisate entstehen, die der Haut einen bräunlichen Farbton verleihen. Diese Reaktion ist nach etwa 4 bis 6 Stunden abgeschlossen. Die so erzielte Bräune ist nicht abwaschbar und wird erst mit der normalen Hautabschuppung entfernt. Diese Farbprodukte besitzen allerdings selbst keine UV-absorbierenden Eigenschaften, sodass bei Sonnenexposition ein zusätzlicher Sonnenschutz (Bekleidung, Hut, UV-Filter) erforderlich wird. Im Gegensatz zu "sonnengebräunter" Haut ist die so gebräunte Haut nicht gegen Sonnenbrand geschützt.

Aufgabe der vorliegenden Erfindung ist es daher, Nachteile des Standes der Technik zu überwinden und Möglichkeiten zu finden, kosmetische und dermatologische Selbstbräunungsformulierungen bereitzustellen, die die natürliche Bräunung der Haut durch gesteigerte Melaninsynthese verstärken und gleichzeitig zu einem besseren Hauteigenschutz bzw. Sonnenschutz, insbesondere gegen UVB-Strahlung, führen.

Es wurde nun überraschenderweise gefunden, dass sich bestimmte Chromen-4-on-Derivate (Chromon-Derivate), als Selbstbräunungswirkstoffe eignen.

Im Sinne der Erfindung wird der Begriff Selbstbräunungswirkstoff synonym zu Selbstbräunungssubstanz oder Selbstbräunersubstanz verwendet.

Gleiche Anwendungen strukturell abweichender Verbindungen sind aus der Literatur bekannt:
DE 10341663 beschreibt Cyclohexen-Derivate zur Anwendung auf der Haut oder dem Haar zur Steigerung der Hautbräunung sowie der Melanin-Synthese in der Haut oder dem Haar.

Ähnliche Anwendungen strukturell verwandter Verbindungen sind aus der Literatur bekannt:
Zubereitungen zur topischen Anwendung, die Chromon-Derivate, wie z.B. Chromon, 7-Hydroxychromon, 7-Methoxychromon, 5,7-Dihydroxy-2-methyl-chromon, 3-Methyl -2-butenyloxy-chromon, 3-Acetyl-5,7-dihydroxy-2-methyl-chromon, 5-hydroxychromon, n-Pentyl-7-methoxychromon-2-carboxylat, n-Undecyl-5-methoxychromon-2-carboxylat, 5-Hydroxy-7-methoxy-2-methyl-chromon, 7-Methoxy-chromon-2-carbonsäure, n-Pentyl-chromon-2-carbonsäure, 5-methoxychromon und Chromon-2-carbonsäure, enthalten, sind aus der japanischen Patentanmeldung JP 05/301813 bekannt. Die Chromon-Derivate wirken als hautverträgliche Tyrosinase-Inhibitoren, welche die Hyperpigmentierung der Haut verringern.

Aus der Japanischen Patentanmeldung JP 09/188608 ist die Verwendung von substituierten Chromon-Derivaten, wie insbesondere 5,7-Dihydroxychromone, 7-Methoxychromone, 5-Hydroxy-7-methoxy-2-methyl-chromon und 5-Hydroxy-2-methyl-chromon, als Wirkstoff gegen graue Haare bekannt. Die Wirkung wird dabei auf die Aktivierung der Farbpigmentbildenden Zellen und die Steigerung der Melanogenese zurückgeführt.

Weiterhin wird in EP 1508327 A1 die Verwendung von bestimmten Chromen-4-on-Derivaten zur Pflege des allgemeinen Zustands der Haut oder Haare, zur Prophylaxe gegen Alterungsprozesse der Haut oder Haare, sowie zur Prophylaxe und Behandlung von Hautkrankheiten offenbart..

Ein erster Gegenstand der vorliegenden Erfindung ist daher die Verwendung von Verbindungen der Formel I 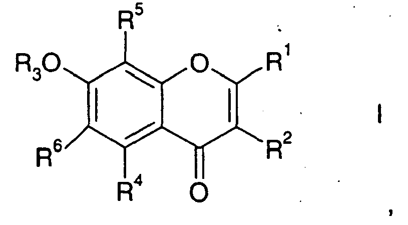 wobei
R¹ und R² gleich oder verschieden sein können und für H, OH, OCH₃, CH₃, CH₂OH oder CH₂OCH₃ stehen,
R³ steht für H oder geradkettige oder verzweigte C₁- bis C₂₀-Alkylgruppen,
R⁴ steht für H oder OR⁸,
R⁵ und R⁶ gleich oder verschieden sein können und ausgewählt sind aus.
- -H, -OH,
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und
R⁸ steht für H oder geradkettige oder verzweigte C₁- bis C₂₀-Alkylgruppen,
mit der Maßgabe, dass R² nicht H bedeutet, wenn R¹ Methyl bedeutet,
als Selbstbräunungssubstanz.

Grundsätzlich sind im Sinne der vorliegenden Erfindung von der Bezeichnung "Verbindung nach Formel I" auch die Salze der jeweiligen Verbindungen nach Formel umfasst. Zu den bevorzugten Salzen gehören dabei insbesondere Alkali- und Erdalkalimetallsalze sowie AmmoniumSalze, insbesondere jedoch Natrium- und Kalium-Salze.

Es ist für die erfindungsgemäße Verwendung bevorzugt, wenn die Verbindungen dadurch gekennzeichnet sind, dass R¹ für CH₂OCH_{3,} CH₂OH oder CH₃ steht.

Im Hinblick auf das gewünschte Eigenschaftsprofil hat es sich weiterhin als bevorzugt erwiesen, wenn die Verbindungen dadurch gekennzeichnet sind, dass R² für H, OH oder OCH₃ steht. Besonders bevorzugt steht R² für OH oder OCH₃. Ganz besonders bevorzugte Selbstbräunersubstanzen sind Verbindungen der Formel I, in denen R² für OCH₃ steht.

Außerdem bevorzugt ist es, wenn die erfindungsgemäß verwendeten Verbindungen dadurch gekennzeichnet sind, dass R³ für H und R⁴ für H oder OH stehen.

Außerdem bevorzugt ist es, wenn die erfindungsgemäß verwendeten Verbindungen dadurch gekennzeichnet sind, dass R⁵ OH oder H bedeutet und R⁶ H bedeutet.

Erfindungsgemäß insbesondere bevorzugt werden Verbindungen nach Formel I als Selbstbräunungssubstanz verwendet, ausgewählt aus den Verbindungen mit den Formeln Ia-Ig:

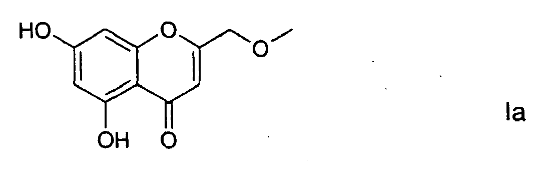

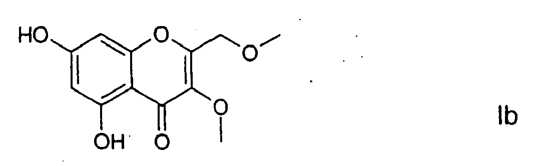

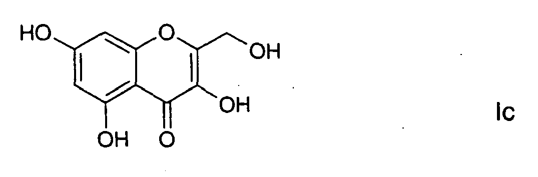

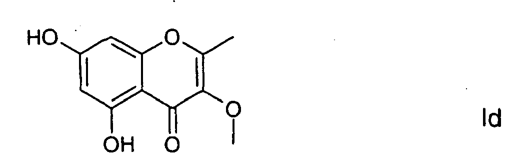

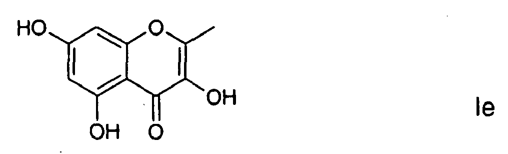

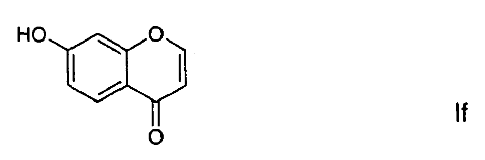

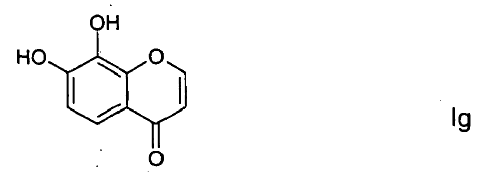

Ganz besonders bevorzugte Selbstbräunungswirkstoffe sind die Verbindungen Id und If.

Gegenstand der Erfindung sind weiterhin die Verbindungen der Formel I 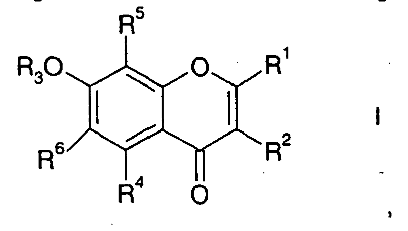 wobei
R¹ CH₂OH oder CH₂OCH₃ bedeutet,
R² OH oder OCH₃ bedeutet,
R³ steht für H oder geradkettige oder verzweigte C₁- bis C₂₀-Alkylgruppen,
R⁴ steht für H oder OR⁸,
R⁵ und R⁶ gleich oder verschieden sein können und ausgewählt sind aus
- -H, -OH,
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und
R⁸ steht für H oder geradkettige oder verzweigte C₁- bis C₂₀-Alkylgruppen.

Geradkettige oder verzweigte Alkylgruppen mit 1 bis 20 C-Atomen, vorzugsweise mit 1, 2, 3, 4, 5 oder 6 C-Atomen, sind beispielsweise Methyl, Ethyl, Isopropyl, n-Propyl, Isobutyl, n-Butyl, tert.-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosanyl, sowie deren Isomere.

Alkenylgruppen sind Gruppen der Summenformel CₙH₂ₙ₋₁ mit n = 3 bis 20.

In einer Erfindungsvariante ist es bevorzugt, wenn R³ in Formel I für H steht.

In einer Erfindungsvariante ist es bevorzugt, wenn R⁴ für H oder OH steht.

In einer Erfindungsvariante ist es bevorzugt, wenn R⁵ für H oder OH steht und R⁶ für H steht.

Bevorzugte neue Verbindungen der Formel I sind die Verbindungen der Formel Ib und Ic:

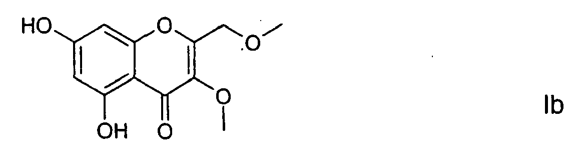

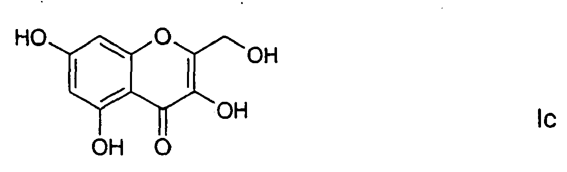

Als Auswahl aus der allgemeinen Formel I, zuvor für die Verwendung definiert, sind die neuen Verbindungen der Formel I bevorzugt als kosmetische Wirkstoffe zu verwenden, insbesondere bevorzugt als Selbstbräunungssubstanzen.

Ein weiterer Gegenstand der Erfindung ist daher eine Zubereitung enthaltend mindestens eine Verbindung der Formel I 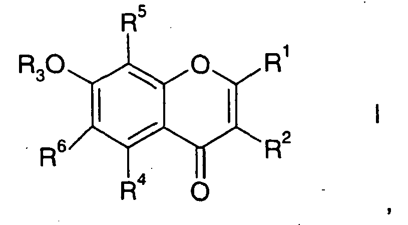 wobei
R¹ CH₂OH oder CH₂OCH₃ bedeutet,
R² OH oder OCH₃ bedeutet,
R³ steht für H oder geradkettige oder verzweigte C₁- bis C₂₀-Alkylgruppen,
R⁴ steht für H oder OR⁸,
R⁵ und R⁶ gleich oder verschieden sein können und ausgewählt sind aus
- -H, -OH,
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und
R⁸ steht für H oder geradkettige oder verzweigte C₁- bis C₂₀-Alkylgruppen und mindestens einen für topische Anwendungen geeigneten Träger.

Ein weiterer Gegenstand der vorliegenden Erfindung ist eine Selbstbräunungszubereitung enthaltend mindestens eine Verbindung der Formel I 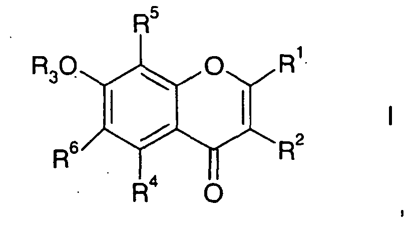 wobei
R¹ und R² gleich oder verschieden sein können und für H, OH, OCH₃, CH₃, CH₂OH oder CH₂OCH₃ stehen,
R³ steht für H oder geradkettige oder verzweigte C₁- bis C₂₀-Alkylgruppen,
R⁴ steht für H oder OR⁸,
R⁵ und R⁶ gleich oder verschieden sein können und ausgewählt sind aus
- -H, -OH,
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und
R⁸ steht für H oder geradkettige oder verzweigte C₁- bis C₂₀-Alkylgruppen,
mit der Maßgabe, dass R² nicht H bedeutet, wenn R¹ Methyl bedeutet und mindestens einen für topische Anwendungen geeigneten Träger.

Erfindungsgemäß bevorzugte Verwendungen der Verbindungen nach Formel I mit Resten wie vorher beschrieben bzw. von Zubereitungen enthaltend mindestens eine Verbindung nach Formel I, wie zuvor beschrieben, sind dabei insbesondere die Verwendung als Selbstbräunungssubstanz und/oder zur Steigerung der Melaninsynthese in der Haut.

Erfindungsgemäß bevorzugte Verwendungen der Verbindungen gemäß Formel la bis Ig bzw. von Zubereitungen enthaltend mindestens eine Verbindung nach Formel la bis Ig sind ebenfalls die Verwendung zur Steigerung der Melaninsynthese in der Haut.

Dabei ist jeweils die Verwendung der Verbindungen nach Formel I, wie zuvor definiert, zur Herstellung von Zubereitungen für die oben angegebenen Verwendungen auch Gegenstand der vorliegenden Erfindung.

Der Gehalt der Verbindungen der Formel I in der Zubereitung beträgt zwischen 0.01 bis 10 Gew.%, vorteilhaft zwischen 0.1 bis 5 Gew%, bezogen auf das Gesamtgewicht der Zubereitung. Dabei bereitet es dem Fachmann keinerlei Schwierigkeiten die Mengen abhängig von der beabsichtigten Wirkung der Zubereitung entsprechend auszuwählen.

Im Sinne der Erfindung wird der Begriff Zubereitung gleichbedeutend mit dem Begriff Formulierung verwendet.

Bei den Zubereitungen handelt es sich dabei üblicherweise entweder um topisch anwendbare Zubereitungen, beispielsweise kosmetische oder dermatologische Formulierungen, oder um Nahrungsmittel bzw. Nahrungsergänzungsmittel. Die Zubereitungen enthalten in diesem Fall einen kosmetisch oder dermatologisch oder Nahrungsmittel-geeigneten Träger und je nach gewünschtem Eigenschaftsprofil optional weitere geeignete Inhaltsstoffe.

Die Verbindungen der Formel I, wie zuvor beschrieben, können in den erfindungsgemäßen Zubereitungen auch in feinteiliger Form dispergiert vorkommen. Dies erzeugt einen slow-release-Effekt, d.h. ein kontrolliertes bzw. verzögertes Eindringen in die Haut bzw. eine kontrollierte Verteilung des Chromen-4-on-Derivats auf oder in der Haut.

Die erfindungsgemäßen Zubereitungen können die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Die kosmetische oder dermatologische Zubereitung bzw. Selbstbräunerzubereitung, wie zuvor beschrieben, kann vorzugsweise mindestens eine weitere Selbstbräunersubstanz enthalten. Dadurch kann der Bräunungseffekt noch verstärkt bzw. verändert werden.

Als Selbstbräunungssubstanzen können unter anderem eingesetzt werden: 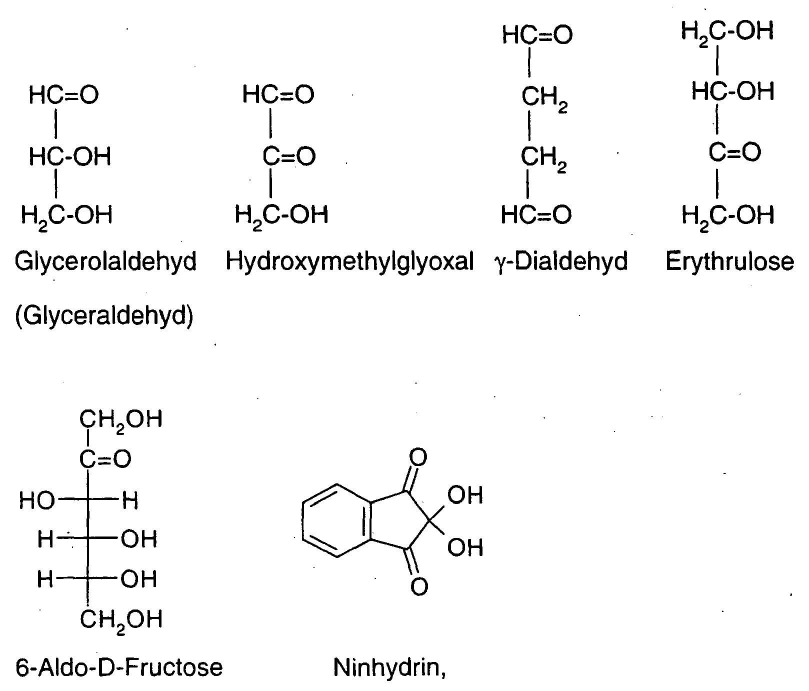 5-Hydroxy-1,4-naphtochinon (Juglon), das aus den Schalen frischer Walnüsse extrahiert wird, 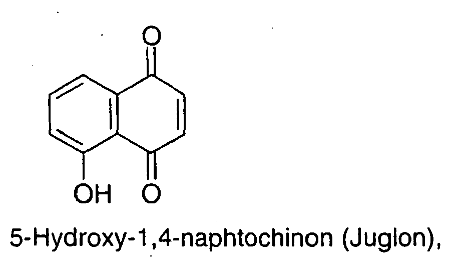 das in den Henna-Blättern vorkommende 2-Hydroxy-1,4-naphtochinon (Lawson) 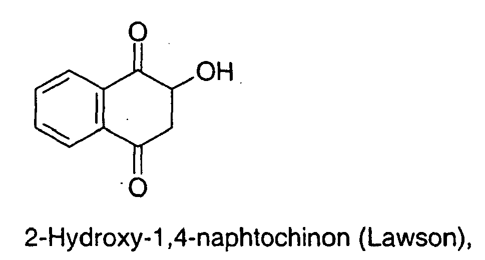 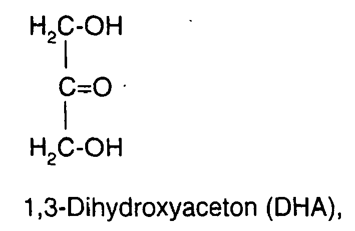 Dihydroxyacetonphosphat, Glyceraldehydphosphat oder Erythrose.

Bevorzugt werden die Selbstbräunersubstanzen 1,3-Diyhydroxyaceton (DHA), Glyceraldehyd, Dihydroxyacetonphosphat, Glyceraldehydphosphat, Erythrose oder 1,3,4-Trihydroxy-2-butanon (Erythrulose) eingesetzt.

Besonders bevorzugt können die Erythrulose oder das 1,3-Dihydroxyaceton (DHA), ein im menschlichen Körper vorkommender dreiwertiger Ketozucker und dessen Derivate enthalten sein. Ganz besonders bevorzugt kann DHA enthalten sein.

Die erfindungsgemäße Zubereitung, welche eine Selbstbräunungssubstanz und ein Chromon der Formel I kombiniert, hat gegenüber einem Selbstbräunungsprodukt ohne Chromon-Zugabe folgende Vorteile:
- Stabilisierung der Selbstbräunungssubstanz gegenüber Sauerstoff (auf der Haut und im Produkt),
- Beschleunigung der Bräunungs-Reaktion,
- Verlängerung der Bräunungs-Reaktion aufgrund der indirekten Bräunungs-Reaktion (UV-freie Bräunungsverlängerung)
- Verstärkung der Bräunungs-Reaktion
- die erzielte Bräunung kommt der natürlichen Bräunung nahe.

In einer weiteren bevorzugten Ausführungsform der vorliegenden Erfindung sind in der Zubereitung ein oder mehrere Antioxidantien und/oder ein oder mehrere Vitamine enthalten.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, trans-Stilbenoxid).

Geeignete Antioxidantien sind auch Verbindungen der allgemeinen Formel 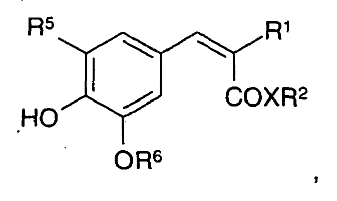 worin
R¹ aus der Gruppe -C(O)CH₃, -CO₂R³, -C(O)NH₂ und -C(O)N(R⁴)₂ ausgewählt werden kann,
X O oder NH,
R² lineares oder verzweigtes Alkyl mit 1 bis 30 C-Atomen,
R³ lineares oder verzweigtes Alkyl mit 1 bis 20 C-Atomen,
R⁴ jeweils ungabhängig voneinander H oder lineares oder verzweigtes Alkyl mit 1 bis 8 C-Atomen,
R⁵ lineares oder verzweigtes Alkyl mit 1 bis 8 C-Atomen oder lineares oder verzweigtes Alkoxy mit 1 bis 8 C-Atomen und
R⁶ lineares oder verzweigtes Alkyl mit 1 bis 8 C-Atomen bedeutet, vorzugsweise Derivate der 2-(4-Hydroxy-3,5-dimethoxybenzyliden)-malonsäure, besonders bevorzugt 2-(4-Hydroxy-3,5-dimethoxybenzyliden)-malonsäure-bis-(2-ethylhexyl)ester (z.B. Okynex^{®} ST.Liquid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit Verbindungen der Formel I in solchen Formulierungen üblicherweise in Verhältnissen im Bereich von 1000:1 bis 1:1000, bevorzugt in Mengen von 100:1 bis 1:100 eingesetzt.

Die erfindungsgemäßen Zubereitungen können als weitere Inhaltsstoffe Vitamine enthalten, wie zuvor beschrieben. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Zubereitungen enthalten, insbesondere bevorzugt Vitamin C und dessen Derivaten, DL-α-Tocopherol, Tocopherol-E-Acetat, Nicotinsäure, Pantothensäure und Biotin. Vitamine werden dabei mit Verbindungen der Formel la-m überlicherweise in Verhältnissen im Bereich von 1000:1 bis 1:1000, bevorzugt in Mengen von 100:1 bis 1:100 eingesetzt.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Radical Biotogy&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Vorteile der erfindungsgemäßen Zubereitungen sind dabei neben der Bräunungswirkung insbesondere auch die antioxidante Wirkung und die gute Hautverträglichkeit. Zusätzlich sind bevorzugte der hier beschriebenen Verbindungen farblos oder nur schwach gefärbt und führen so nicht oder nur in geringer Weise zu Verfärbungen der Zubereitungen. Von Vorteil ist insbesondere das besondere Wirkprofil der Verbindungen nach Formel I, welches sich im DPPH-Assay in einer hohen Kapazität Radikale zu fangen (EC₅₀), einer zeitverzögerten Wirkung (T_{EC50} > 120 min) und damit einer mittleren bis hohen antiradikalischen Effizienz (AE) äußert. Zudem vereinigen die Verbindungen nach Formel I im Molekül antioxidative Eigenschaften mit UV-Absorption im UV-B-Bereich.

Damit die Verbindungen der Formel I ihre positive Wirkung auf die Haut besonders gut entwickeln können, kann es bevorzugt sein die Verbindungen der Formel I, wie zuvor beschrieben, in tiefere Hautschichten eindringen zu lassen. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar. Die Zubereitung wird dann beispielsweise so gestaltet, dass sie für eine orale Gabe geeignet ist.

In einer weiteren Erfindungsvariante kann es von Vorteil sein, dass in den beschriebenen Zubereitungen neben den Verbindungen der Formel I auch UV-Filter enthalten sind.

Prinzipiell kommen alle UV-Filter für eine Kombination mit den Verbindungen der Formel in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.
Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),
Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),
Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),
Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000);
Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),
4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),
Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie
- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicycto-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin (z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1-8 Gew.-%, in kosmetische Formulierungen eingearbeitet.

Weitere geeignete organische UV-Filter sind z.B.
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole^{®}),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),
- α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n = 60) (CAS-Nr. 207574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB).

Weitere geeignete UV-Filter sind auch Methoxyflavone ensprechend der älteren Deutschen Patentanmeldung DE 10232595.2.

Organische UV-Filter werden in der Regel in einer Menge von 0,5 bis 20 Gewichtsprozent, vorzugsweise 1-15 Gew.-%, in kosmetische Formulierungen eingearbeitet.

Als anorganische UV-Filter sind solche aus der Gruppe der Titandioxide wie z.B. gecoatetes Titandioxid (z.B. Eusolex® T-2000, Eusoiex^{®}T-AQUA), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2-10 %, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-methoxy-benzophenon, Methoxyzimtsäureoctylester, 3,3,5-Trimethyl-cyclo-hexylsali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanol-aminsalze.

Durch Kombination von einer oder mehrerer Verbindungen der Formel I mit weiteren UV-Filtern kann die Schutzwirkung gegen schädliche Einwirkungen der UV-Strahlung optimiert werden.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:
- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

In den Zubereitungen, wie zuvor beschrieben, können auch weitere hautschonende oder hautpflegende Wirkstoffe enthalten sein. Dabei kann es sich prinzipiell um alle den Fachmann bekannten Wirkstoffe handeln.

Besonders bevorzugte Wirkstoffe sind in einer Ausführungsform der vorliegenden Erfindung Pyrimidincarbonsäuren und/oder Aryloxime.

Pyrimidincarbonsäuren kommen in halophilen Mikroorganismen vor und spielen bei der Osmoregulation dieser Organismen eine Rolle (E. A. Galinski et al., Eur. J. Biochem., 149 (1985) Seite 135-139). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-4116123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriäsis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben der Verbindung der Formel la bis Ig zusätzlich eine Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

Alle Verbindungen oder Komponenten, die in den Zubereitungen verwendet werden können, sind entweder bekannt und käuflich erwerbbar oder können nach bekannten Verfahren synthetisiert werden. Die Herstellung der neuen Verbindungen nach der Formeln 1, wie zuvor beschriebn wird nachfolgend beschrieben.

Die mindestens eine Verbindung der Formel I kann in der üblichen Weise in kosmetische oder dermatologische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Stabilisatoren, Lösungsvermittler, Färbemittel oder Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettlkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂-₁₅-Alkylbenzoat, Capryl-Caprinsäure-triglycerid, Dicaprylether.

Besonders vorteilhaft sind Mischungen aus C₁₂-₁₅-Alkylbenzoat und 2-Ethylhexylisostearat, Mischungen aus C₁₂-₁₅-Alkylbenzoat und Isotridecylisononanoat sowie Mischungen aus C₁₂-₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearen Silikonölen aufweisen oder vollständig aus solchen Ölen bestehen, wobei allerdings bevorzugt wird, außer dem- Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft. Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren^{®} 1200 (Henkel KGaA), Oramix^{®} NS 10 (Seppic).

Die Acyllactylate werden ihrerseits vorteilhaft gewählt aus der Gruppe der Substanzen, welche sich durch die Strukturformel 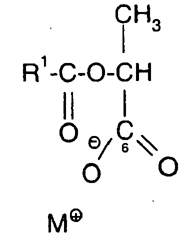 auszeichnen, wobei R¹ einen verzweigten oder unverzweigten Alkylrest mit 1 bis 30 Kohlenstoffatomen bedeutet und M⁺ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic^{®} ISL von der Gesellschaft American Ingredients Company.

Die Betaine werden vorteilhaft gewählt aus der Gruppe der Substanzen, welche sich durch die Strukturformel 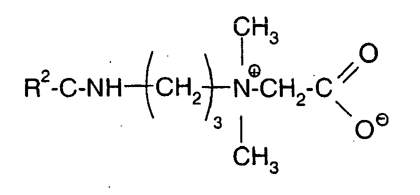 auszeichnen, wobei R² einen verzweigten oder unverzeigten Alkylrest mit 1 bis 30 Kohlenstoffatomen bedeutet.

Insbesondere vorteilhaft bedeutet R² einen verzweigten oder unverzweigten Alkylrest mit 6 bis 12 Kohlenstoffatomen.

Vorteilhaft ist beispielsweise Capramidopropylbetain, beispielsweise das Produkt Tego^{®} Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol^{®} Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise auf die Haut und/oder die Haare, bevorzugt auf die Haut, in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)-stearylether (Steareth-17),Polyethylenglycol(18)stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)-isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)-isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)-isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)-cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(1 6)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Weitere Gegenstände der vorliegenden Erfindung sind ein Verfahren zur Herstellung einer Zubereitung, welches dadurch gekennzeichnet ist, dass mindestens eine Verbindung der Formel I mit Resten wie oben beschrieben vorzugsweise mit einem kosmetisch oder dermatologisch geeigneten Träger oder mit einem für Nahrungsmittel geeigneten Träger vermischt wird, und die Verwendung einer Verbindung der Formel I zur Herstellung einer Zubereitung.

Die erfindungsgemäßen Zubereitungen können dabei mit Hilfe von Techniken hergestellt werden, die dem Fachmann wohl bekannt sind.

Das Vermischen kann ein Lösen, Emulgieren oder Dispergieren der mindestens einen Verbindung der Formel I in dem Träger zur Folge haben.

Die Verbindungen der Formel I, wie zuvor für die erfindungsgemäße Verwendung definiert, können durch Cyclisierung eines entsprechend substituierten o-Hydroxyacetophenons mit einem Anhydrid oder mit einem Acylchlorid unter basischen Bedingungen erhalten werden. Anschließend können die Acylgruppen, d.h. zum einen O-Acylgruppen, die durch Reaktion der freien Hydroxygruppe mit dem Anhydrid oder Acylchlorid entstanden sind, zum anderen C-Acylgruppen in Position 3 des Chromonsystems, entfernt werden. Dabei kann die Umsetzung analog zu Kelly, T; Kim M.H.; J. Org. Chem. 1992, 57, 1593-97 erfolgen.

Alternativ werden die freien Hydroxy-Gruppen acyliert. Anschließend folgt eine Baker-Venkatamaran-Umlagerung in basischen Bedingungen mit anschließendem Ringschluss unter sauren Bedingungen. Entsprechende Umsetzungen, deren Adaption an die hier gewünschten Verbindungen dem Fachmann keinerlei Probleme bereitet, sind aus der Patentanmeldung WO 2002/060889 bekannt.

Durch übliche Umsetzungen an dem Ringsystem oder Derivatisierung der funktionellen Gruppen können weitere Derivate gemäß Formel I erhalten werden. Die dazu notwendigen Reaktionsbedingung für solche Reaktionen, wie beispielsweise Oxidationen, Reduktionen, Umesterungen, Veretherungen, findet ein Fachmann für derartige Synthesen problemlos in der allgemein zugänglichen Literatur zu organischen Reaktionen.

Ein weiterer Gegenstand der Erfindung ist auch ein Verfahren zur Herstellung der Verbindungen der Formel I, 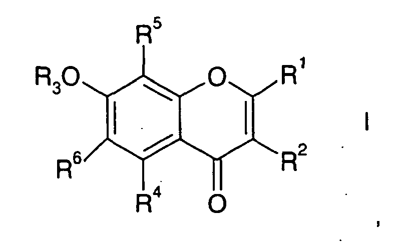 wobei
R¹ CH₂OH oder CH₂OCH₃.
R² OH oder OCH₃ bedeuten,
R³ steht für H oder geradkettige oder verzweigte C₁- bis C₂₀-Alkylgruppen,
R⁴ steht für H oder OR⁸,
R⁵ und R⁶ gleich oder verschieden sein können und ausgewählt sind aus
- -H, -OH,
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und
R⁸ steht für H oder geradkettige oder verzweigte C₁- bis C₂₀-Alkylgruppen, dadurch gekennzeichnet, dass
ein o-Hydroxyacetophenon der Formel II 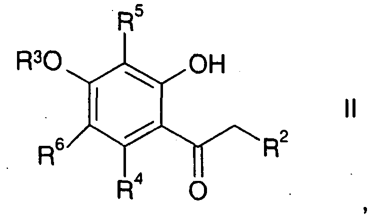 wobei die Substituenten R² bis R⁶ eine der zuvor beschriebenen oder bevorzugten Bedeutungen haben,
mit Methoxyacetylchlorid unter basischen Bedingungen cyclisiert wird
und gegebenenfalls die vorhandenen Methylether gespalten werden.

Bevorzugt findet die Cyclisierung in einem Lösungsmittel statt, ausgewählt aus der Gruppe Tetrahydrofuran, Diethylether, Diisopropylether, Methylter-butylether oder 1,4-Dioxan.

Die Cyclisierung erfolgt vorzugsweise unter Inertgasbedingungen. Vorteilhaft wird K₂CO₃ oder ein anderes Reagenz aus der Gruppe KO-tBu (Kaliumtertbutoxid), LiOH, KOH. oder DBU in Pyridin bei Temperaturen zwischen -10°C und 35°C, bevorzugt bei Raumtemperatur, eingebracht. Die eigentliche Reäktionstemperatur liegt zwischen -10 und 175°C, bevorzugt zwischen 35 und 100°C. Besonders bevorzugt erfolgt die Umsetzung bei 50°C oder 66°C.

Die Verbindungen der Formel I, in denen R¹ CH₂-OH und R² OH bedeuten, werden vorzugsweise durch anschließende Etherspaltung aus den Verbindungen der Formel I hergestellt, in denen R¹ CH₂-OCH₃ und R² OCH₃ bedeuten.

Es wurde auch festgestellt, dass Verbindungen der Formel I, wie zuvor beschrieben, stabilisierend auf die Zubereitung wirken können. Bei der Verwendung in entsprechenden Produkten bleiben diese daher auch länger stabil und verändern ihr Aussehen nicht. Insbesondere bleibt auch bei längendauernder Anwendung bzw. längerer Lagerung die Wirksamkeit der Inhaltsstoffe, z.B. Vitamine, erhalten. Dies ist unter anderem besonders vorteilhaft bei Zusammensetzungen zum Schutz der Haut gegen die Einwirkung von UV-Strahlen, da diese Kosmetika besonders hohen Belastungen durch die UV-Strahlung ausgesetzt sind.

Die positiven Wirkungen von Verbindungen der Formel I ergeben deren besondere Eignung zur Verwendung in kosmetischen oder pharmazeutischen Zubereitungen, insbesondere in kosmetischen Zubereitungen.

Desweiteren wurde gefunden, dass die Zwischenstufen bzw. Vorstufen (wie z.B. 5,7-Dihydroxy-3-(2-methoxy-acetyl)-2-methoxy-methyl-1-benzopyran-4-on (Verbindung IIa)) der erfindungsgemäßen Verbindungen nach Formel I ebenfalls eine bräunende Aktivität aufweisen und somit auch als Bräunungsmittel eingesetzt werden können.

Ebenso positiv sind die Eigenschaften von Verbindungen mit der Formel I zu werten für eine Verwendung in Nahrungsmitteln oder als Nahrungsergänzungsmittel oder als "functional food". Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit einer oder mehreren Verbindungen der Formel I angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". (Die Nahrungsmittel können fest sein aber auch flüssig, also als Getränk vorliegen). Weitere Gegenstände der vorliegenden Erfindung sind dementsprechend die Verwendung einer Verbindung nach Formel I als Nahrungsmittelzusatz für die Human- oder Tierernährung sowie Zubereitungen, die Nahrungsmittel oder Nahrungsergänzungsmittel sind und entsprechende Träger enthalten.

Nahrungsmittel, die nach der vorliegenden Erfindung mit einer oder mehreren Verbindungen der Formel I angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensoße, Tomatenpüree usw. Weitere Beispiele für Nahrungsmittel, die nach der vorliegenden Erfindung mit einer oder mehreren Verbindungen der Formel Ia bis Ig angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit einer oder mehreren Verbindungen der Formel I angereichert zu werden, beispielsweise Multivitaminpräparate, Mineralstoffmischungen oder gezuckerter Saft. Als weitere Beispiele für Nahrungsmittel, die nach der vorliegenden Erfindung mit einer oder mehreren Verbindungen der Formel I angereichert werden können, seien Nahrungsmittelzubereitungen, beispielsweise zubereitete Cerealien, Gebäck, Mischgetränke, speziell für Kinder zubereitete Nahrungsmittel, wie Joghurt, Diätnahrungsmittel, kalorienarme Nahrungsmittel oder Tierfutter, genannt.

Die Nahrungsmittel, die nach der vorliegenden Erfindung mit einer oder mehreren Verbindungen der Formel I angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die Nahrungsmittel, die nach der vorliegenden Erfindung mit einer oder mehreren Verbindungen der Formel I angereichert werden können, werden vorzugsweise oral angewendet, z.B. in Form von Speisen, Pillen, Tabletten, Kapseln, Pulver, Sirup, Lösungen oder Suspensionen.

Die mit einer oder mehreren Verbindungen der Formel I angereicherten erfindungsgemäßen Nahrungsmittel können mit Hilfe von Techniken hergestellt werden, die dem Fachmann wohl bekannt sind.

Durch ihre Wirkung eignen sich Verbindungen der Formel I auch als Arzneimittelinhaltsstoff.

Im folgenden wird die Erfindung anhand von Beispielen näher erläutert. Die Erfindung ist im gesamten beanspruchten Bereich ausführbar und nicht auf die hier genannten Beispiele beschränkt.

### Beispiele

### Beispiel 1

### - Herstellung von 5,7-dihydroxy-2-methoxymethylchromen-4-on (Ia)

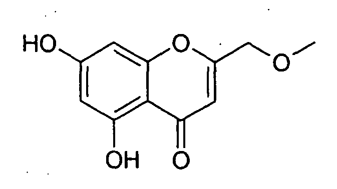

### - Herstellung der Vorstufe 5,7-Dihydroxy-3-(2-methoxy-acetyl)-2-methoxy-methyl-1-benzopyran-4-on (IIa)

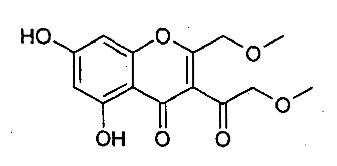

2,4,6-Trihydroxyacetophenon (10 g, 53.7 mmol) werden in 100 ml THF (Tetrahydrofuran) bei Raumtemperatur (RT) unter Inertgas und Rühren gelöst. K₂CO₃ (30 g, 217.1 mmol) wird zugefügt und die gelbe Suspension wird auf 50°C erhitzt und 30 min. lang gerührt. Methoxyacetylchlorid (10.3 mL, 112.8 mmol) wird tropfenweise zu der warmen Suspension hinzugefügt und noch 6 unter Rückfluss, d.h bei 66°C, erhitzt. Die Lösung wird abgekühlt und in Eiswasser geschüttet. HCl (32%) wird zum Ansäuern hinzugefügt und dann die Lösung mit Ethylacetat extrahiert. Die organische Phase wird über Na₂SO₄ getrocknet, filtriert und zu einem Öl eingedampft, welches dann kristallisiert. Die Kristalle werden anschließend mit Dichlormethan gewaschen.
Ausbeute: 7,5 g (46%)
El-MS: *m*/*z*: 294 M⁺

¹H NMR (250 MHz, DMSO): *δ* (ppm) = 3.32 (s, 3H, CH₃ of 2-methoxymethyl); 3.34 (s, 3H, CH₃ of 3-methoxyacetyl); 4.39 (s, 2H, CH₂ of 2-methoxymethyl); 4.46 (s, 2H, CH₂ of 3-methoxyacetyl); 6.25 (d, 1H, *J*=2.0Hz. H-8); 6.39 (d, 1H, *J*=2.0 Hz, H-6); 11.05 (s, 1H, OH-7); 12.21 (s, 1H, OH-5).

¹³C NMR (75 MHz, DMSO, Protonen entkoppelt): *δ* (ppm) = 58.5 (CH₃); 58.6 (CH₃); 68.9 (CH₂); 77.7 (CH₂); 94.1 (CH); 99.5 (CH); 103.2 (Cquat.); 119.5 (Cquat.); 156.7 (Cquat.); 161.4 (Cquat.); 165.0 (Cquat.); 165.3 (Cquat.); 179.2 (Cquat.); 198.8 (Cquat.).

HR-MS: (C₁₄H₁₄O₇) Ber.: 294.0739; Gefunden: 294.0736 ± 1.1 ppm.

### - Herstellung von 5,7-Dihydroxy-2-methoxymethyl-1-benzopyran-4-on (la)

5,7-Dihydroxy-3-(2-methoxy-acetyl)-2-methoxymethyl-chromon **(IIa)** (1 g, 3.4 mmol) wird in 200 ml Wasser suspendiert. K₂CO₃ (1.41 g, 10.2 mmol) wird bei RT unter Rühren hinzugefügt. Die Suspension wird 1 h unter Rückfluss gekocht und dann die gelbe Lösung wieder auf RT abgekühlt. Eis wird zugegeben und die Lösung mit 32% HCl auf pH=4 eingestellt. Eine weißes Präzipitat entsteht, welches filtriert und im Vakuum bei 40°C getrocknet wird. Der Feststoff wird aus Dichlormethan umkristallisiert um ein weißes Produkt zu erhalten.
Ausbeute: 550mg (71%)
MP: 180°C
EI-MS: *m*/*z*: 222 M⁺

¹H NMR (250 MHz, DMSO): *δ* (ppm) = 3.39 (s, 3H, CH₃); 4.37 (s, 2H, CH₂); 6.22 (d, 1H, *J*=1.75 Hz, H-8); 6.26 (s, 1H, H-3); 6.37 (d, 1H, *J*=1.75 Hz, H-6); 10.85 (s, 1H, OH-7), 12.65 (s, 1H, OH-5).

¹³C NMR (75 MHz, DMSO, Protonen entkoppelt): δ (ppm) = 58.3 (CH₃); 69.6 (CH₂); 93.8 (CH); 98.9 (CH); 103.9 (Cquat.); 107.3 (CH); 157.5 (Cquat.); 161.5 (Cquat.); 164.3 (Cquat.); 166.0 (Cquat.); 181.5 (Cquat.).

HR-MS: (C₁₁H₁₀O₅) Ber.: 222.0528; Gefunden: 222.0524 ± 1.9 ppm.

### Beispiel 2

### - Herstellung von 5,7-dihydroxy-3-methoxy-2-methoxymethyl-chromen-4-on (Ib)

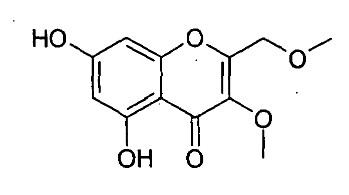

### - Herstellung der Vorstufe 2-Methoxy-1-(2,4,6-trihydroxy-phenyl)-ethanon (IIb)

### (Friedel and Crafts)

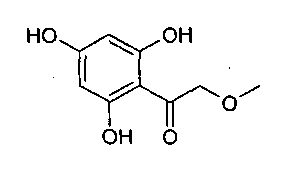

Phloroglucinol (1.3 g, 10 mmol) wird in trockenem Ether (50 ml) unter Inertgas gelöst. Methoxyacetylchlorid (1.03 mL, 11.0 mmol) wird unter Rühren bei RT zugegeben und dann auf 0°C gekühlt bevor die Zugabe von AlCl₃ (5.47 g, 41 mmol) erfolgt. Es wird über Nacht gerührt. Die Reaktionsmischung wird in Eis gegossen und dann 32 % HCl zugefügt zur pH-Einstellung von 5-6. Die Lösung wird mit Diethylether extrahiert und die vereinigten Extrakte mit Wasser gewaschen und mit Na₂SO₄ getrocknet und das Lösungsmittel unter verminderten Druck abgedampft um ein weißes Produkt zu erhalten, welches aus Methanol umkristallisiert wird.
Ausbeute: 1.25 g (63%)
EI-MS: *m*/*z*: 198 M⁺

¹H NMR (250 MHz. DMSO): δ(ppm) = 3.33 (s, 3H, CH₃); 4.58 (s, 2H, CH₂); 5.82 (s, 1H, H-3 and H-5); 10.40 (bs, 1H, OH-4); 12.13 (s, 2H, OH-2 and OH-6).

¹³C NMR (75 MHz, DMSO, Protonen entkoppelt): δ (ppm) = 58.4 (CH₃), 77.0 (CH₂), 94.5 (CH), 102.4 (Cquat.), 163.9 (Cquat.), 164.8 (Cquat.), 201.2 (Cquat.).

HR-MS: (C₉H₁₀O₅) Ber.: 198.0528; Gefunden: 198.0530 ± 1.3 ppm.

### 5,7-Dihydroxy-3-methoxy-2-methoxymethyl-1-benzopyran-4-on (Ib)

2-Methoxy-1-(2,4,6-trihydroxy-phenyl)-ethanon (IIb) (10g, 50.46 mmol) wird in THF (250 mL) bei RT unter Rühren und Inertgas gelöst. K₂CO₃ (34.87 g, 252.3 mmol) wird zugegeben und die gelbe Suspension wird gerührt und auf 50°C für 1 h erhitzt. Methoxyacetylchlorid (9.70 mL, 106 mmol) wird tropfenweise hinzugefügt. Es wird noch 4 h gerührt. Anschließend wird die Lösung abgekühlt und in Eiswasser geschüttet. HCl (32%) wird zur Neutralisation (pH 6) zugefügt welches dann mit Ethylacetat extrahiert wird. Die organische Phase wird mit Na₂SO₄ getrocknet, filtriert, und eingeengt um ein weiß-oranges Produkt zu erhalten. Umkristallisation erfolgt aus Dichlormethan.
Ausbeute: 4.2g (33%)
MP: 173-174°C
EI-MS: *m*/*z* 252 M⁺

¹H NMR (250 MHz, DMSO): *δ* (ppm) = 3.36 (s, 3H, CH₃ of 2-methoxymethyl); 3.81 (s, 3H, CH₃ of 3-methoxy); 4.45 (s, 2H, CH₂); 6.21 (d, 1H, *J*=2.0 Hz, H-8); 6.36 (d, 1H, *J*=2.0 Hz, H-6); 10.88 (s, 1H, OH-7); 12.44 (s, 1H, OH-5).

¹³C NMR (75 MHz, DMSO, Protonen entkoppelt): δ (ppm) = 58.1 (CH₃); 60.8 (CH₃); 65.6 (CH₂); 93.7 (CH); 98.7 (CH); 104.6 (Cquat.); 139.6 (Cquat.); 152.7 (Cquat.); 161.4 (Cquat.); 164.3 (Cquat.); 177.5 (Cquat.).

HRMS: (C₁₂H₁₂O₆) Ber.: 252.0633; Gefunden: 252.0636 ± 1.2 ppm

### Beispiel 3

### - Herstellung von 2-Hydroxymethyl-3,5,7-trihydroxymethyl-chromen-4-on (Ic)

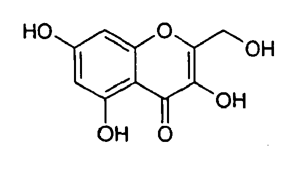

5,7-Dihydroxy-3-methoxy-2-methoxymethyl-chromon (3 g, 11.9 mmol) wird bei RT unter Rühren und Inertgas in Methylenchlorid (50 ml) gelöst. Anschließend wird die Suspension vor der tropfenweisen Zugabe von BBr₃ (3.39 ml, 35.7 mmol) auf 0°C gekühlt. Die gelbe Suspension wird noch 1 h bei RT gerührt. Das gelbe Produkt wird filtriert und mit 32% HCl saurem Wasser (pH 3-4) gewaschen. Der gelbe Feststoff wird im Vakuum bei 40°C getrocknet und anschließend durch Silica-Gelchromatographie mit Hilfe einer Lösung aus Methylenchlorid und MeOH (Methanol) (95/5) gereinigt.
Ausbeute: 1.8 g (48%)
EI-MS: *mlz:* 224 M⁺

¹H NMR (250 MHz, DMSO): *δ* (ppm) = 4.48 (d, 2H, *J*=2.75 Hz, CH₂); 5.47 (t, 1H, *J*=5.5 Hz, OH on CH₂OH); 6.17 (d, 1H, *J*=1.0 Hz, H-8); 6.33 (d, 1H, *J*=1.0 Hz, H-6); 9.09 (s, 1H, OH-3); 10.77 (bs, 1H, OH-7); 12.44 (s, 1H, OH-5).

¹³C NMR (75 MHz, DMSO, Protonen entkoppelt): δ (ppm) = 55.3 (CH₂); 93.3 (CH); 98.1 (CH); 103.6 (Cquat.); 135.9 (Cquat.); 151.6 (Cquat.); 156.6 (Cquat.); 160.9 (Cquat.); 163.9 (Cquat.); 176.3 (Cquat.).

HR-MS: (C₁₀H₈O₆) Ber.: 224.0320; Gefunden: 224.0323 ± 1.0 ppm.

### Beispiel 4

### - Herstellung von 5,7dihydroxy-3-methoxy-2-methyl-chromen-4-on (1d)

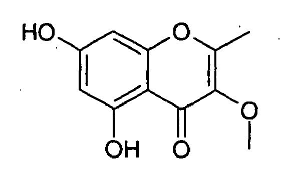

2-Methoxy-1-(2,4,6-trihydroxy-phenyl)-ethanon (5 g, 25.2 mmol) wird in 150 ml THF bei RT und Inertgas unter Rühren gelöst. K₂CO₃ (10.46 g, 65.7 mmol) wird zugesetzt und die gelbe Suspension für 30 min auf 50°C erhitzt. Es wird Acetylchlorid (3.78 mL, 53 mmol) tropfenweise zur warmen Suspension zugegeben und für 20 h unter Rückfluss, d.h. bei 66°C, erhitzt. Anschließend wird die Lösung auf RT abgekühlt und in Eiswasser geschüttet. Es wird mit HCl (32%) neutralisiert (pH 7-8) und dann mit Ethylacetat extrahiert. Die organische Phase wird mit Na₂SO₄ getrocknet, filtriert, und eingeengt. Umkristallisation erfolgt aus Dichlormethan.
Ausbeute: 2.8 g (50%)
EI-MS: *m*/*z*: 222 M⁺

¹H NMR (250 MHz, DMSO): *δ* (ppm) = 2.37 (s, 3H, CH₃-2); 3.75 (s, 3H, OCH₃); 6.17 (d, 1H, *J*=2.0 Hz, H-8); 6.32 (d, 1H, *J*=2.0 Hz, H-6); 10.70 (bs, 1H, OH-7); 12.60 (s, 1H, OH-5).

¹³C NMR (75 MHz, DMSO, Protonen entkoppelt): *δ* (ppm) = 15.0 (CH₃); 60.0 (CH₃); 93.5 (CH); 98.5 (CH); 104.3 (Cquat.); 156.6 (Cquat.); 160.0 (Cquat.); 161.3 (Cquat.); 163.9 (Cquat.); 177.0 (Cquat.).

HR-MS: (C₁₁H₁₀O₅) Ber.: 222.0528; Gefunden: 222.0524 ± 1.9 ppm.

### Beispiel 5

### - Herstellung von 2-Methyl-3,5,7-trihydroxychromen-4-on (Ie)

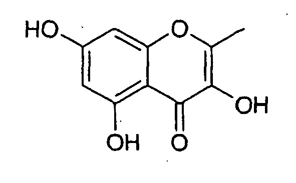

5,7-Dihydroxy-3-methoxy-2-methyl-chromon (1g, 4.5 mmol) wird in 10 ml Methylenchlorid unter Rühren bei RT und Inertgas suspendiert. Es wird auf 0°C gekühlt, bevor BBr₃ tropfenweise zugegeben wird. Es wird noch 1 h bei RT gerührt und das gelbe Produkt filtriert und mit einer wässrigen Lösung von HCl (0.05M) gewaschen. Der Feststoff wird bei 40°C im Vakuum getrocknet und aus Dichlormethan umkristallisiert (weißer Feststoff).
Ausbeute: 800 mg (91%)
EI-MS: *m*/*z*: 208 M⁺

¹H NMR (250 MHz, DMSO): *δ* (ppm) = 2.33 (s, 3H, CH₃); 6.15 (d, 1H, *J*=1.0 Hz, H-8); 6.30 (d, 1H, *J*=1.0Hz, H-6); 8.84 (bs, 1H, OH-3); 10.68 (bs, 1H, OH-7); 12.54 (s, 1H, OH-5).

¹³C NMR (75 MHz, DMSO, Protonen entkoppelt): δ (ppm) = 14.7 (CH₃); 93.2 (CH); 98.1 (CH); 103.4 (Cquat.); 136.0 (Cquat.); 151.1 (Cquat.); 156.4 (Cquat.); 160.9 (Cquat.); 163.4 (Cquat.); 175.4 (Cquat.); 177.0 (Cquat.).

HR-MS: (C₁₀H₈O₅) Ber.: 208.0371; Gefunden: 208.0375 ± 1.8 ppm.

### Beispiel 6

### - Herstellung von 7-Hydroxychromen-4-on (If)

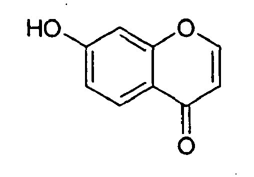

3-Formyl-7-hydroxy-chromon (300 mg, 1.58 mmol) wird unter Rühren in Wasser (80ml) bei 50°C suspendiert. K₂CO₃ (654 mg, 4.73 mmol) wird zugegeben und 30 min unter Rückfluss gekocht. Die braune Lösung wird auf Raumtemperatur bzw. dann auf 0°C abgekühlt und 32%ige HCl zur. Neutralisation (pH 6-7) zugetropft. Es entsteht ein leicht-brauner Feststoff, der filtriert und im Vakuum bei 40°C getrocknet wird. Die Mutterlauge wird mit Ethylacetat extrahiert und die organischen Phasen mit Na₂SO₄ getrocknet, filtriert und eingeengt, so dass eine tief-braune Verbindung entsteht. Es wird durch Silica-Gelchromatographie mit einer Mischung aus Methylenchlorid und MeOH (97/3) gereinigt, wobei ein leicht gelber Feststoff entsteht.
Ausbeute: 90 mg (35%)
EI-MS: *m*/*z*: 162 M⁺

¹H NMR (250 MHz, DMSO): *δ* (ppm) = 6.20 (d, 1H, *J*=5.75 Hz, H-3); 6.84 (d, 1H, *J*=2.0 Hz, H-8); 6.91 (dd, 1H, *J*=8.5 Hz, *J*=2.0Hz, H-6); 7.87 (d, 1H, *J*=8.5 Hz, H-5); 8.13 (d, 1H, *J*=5.75 Hz, H-2); 10.73 (bs, 1H, OH).

¹³C NMR (75 MHz, DMSO, Protonen entkoppelt): δ (ppm) = 102.3 (CH); 111.9 (CH); 115.0 (CH); 117.0 (Cquat.); 126.6 (CH); 156.0 (CH); 157.7 (Cquat.); 162.5 (CH); 175.5 (Cquat.).

HR-MS: (C₉H₆O₃) Ber.: 162.0317; Gefunden: 162.0316 ± 0.1 ppm.

### Beispiel 7

### - Herstellung von 7,8-Dihydroxychromen-4-on (Ig)

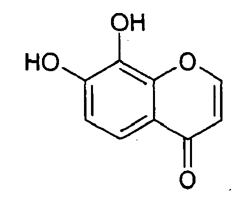

3-Formyl-7,8-dihydroxy-chromon (500 mg, 2.42 mmol) wird in Wasser (100 mL) unter Rühren bei 50°C suspendiert. K₂CO₃ (1.0 g, 7.27 mmol) wird zugegeben und die Suspension 30 min unter Rückfluss gekocht. Die braune Lösung wird auf RT bzw. dann auf 0°C abgekühlt und 32%ige HCl zur Neutralisation (pH 6-7) zugetropft. Es entsteht ein tief-brauner Feststoff, der filtriert und im Vakuum bei 40°C getrocknet wird. Die Mutterlauge wird mit Ethylacetat extrahiert und die organischen Phasen mit Na₂SO₄ getrocknet, filtriert und eingeengt, so dass eine tief-braune Verbindung entsteht. Es wird durch Silica-Gelchromatographie mit einer Mischung aus Methylenchlorid und MeOH (95/5) gereinigt, wobei ein leicht gelber Feststoff entsteht.
Ausbeute: 20 mg (47%)
EI-MS: *m*/*z* 178 M⁺

¹H NMR (250 MHz, DMSO): *δ* (ppm) = 6.17 (d, 1H, *J*=5.75 Hz, H-3); 6.93 (d, 1H, *J*=8.5 Hz H-6); 7.37 (d, 1H, *J*=8.5 Hz, H-5); 8.17 (d, 1H, *J*=5.75 Hz, H-2); 9.36 (bs, 1H, OH-8); 10.25 (bs, 1H, OH-7).

¹³C NMR (75 MHz, DMSO, Protonen entkoppelt): δ (ppm) = 111.3 (CH); 113.9 (CH); 115.0 (CH); 117.8 (Cquat.); 133.0 (Cquat.); 146.9 (Cquat.); 150.1 (Cquat.); 155.8 (CH); 176.0 (Cquat.).

HR-MS: (C₉H₆O₄) Ber.: 178.0266; Gefunden: 178.0264 ± 0.8 ppm.

### Beispiel 8

### Melanogenese-Assay:

Die Wirkung der Verbindungen nach Formel I als Bräunungsmittel wird geprüft durch die Fähigkeit die Melanin-Synthese zu erhöhen. Als Referenz wird Kojicsäure verwendet, die auf die Melaninsynthese wirkt und eine mögliche Braunfärbung der Haut verhindert. Aus der Tab. 1 wird ersichtlich, dass sich die erfindungsgemäßen Verbindungen 1a bis 1g dadurch auszeichnen, dass sie -nach topischer Anwendung- in der Haut die Bildung von hauteigenen Pigmenten induzieren, die Melaninsynthese in der Haut steigern und auf diese Weise eine verstärkte Bräunung der Haut erzeugen. Sie sind gesundheitlich unbedenklich, nicht reizend und leicht zu handhaben, und der resultierende Farbton entspricht naturgemäß dem der natürlichen gesunden Hautfarbe. Die erhaltene Bräunung ist, da sie der natürlichen Bräunung entspricht, lichtecht und nicht abwaschbar.

Dazu werden primäre normale menschliche Melanozyten (R6-NHEM-2)* in 24 Mikrotiterplatten (96er, 120.000 Zellen pro Tube) kultiviert. Zwei zusätzliche Platten werden durchgeführt, eine zur Evaluierung der Lebensfähigkeit der Zellen (Standard MTT assay) und eine zweite für die Proteinuntersuchung (unter Verwendung eines kolorimetrischen Standardprotokolls). Nach 50% Zusammenfluss wird das Kulturmedium gegen frisches ersetzt, welches entweder die Kontrolllösung (KA oder IBMX) oder die zu untersuchenden 7 Lösungen enthält. Jeder Versuch wurde 3 mal wiederholt. Die Zellen werden bei 37°C (und 5% CO₂) 240 Stunden inkubiert, wobei alle 3 Tage das Medium gewechselt wird. Nach der Inkubation werden Monolayers gespült, die Zellen werden zerstört und Melanin mittels einer 0.5 M NaOH-Lösung extrahiert.
Die optische Dichte (OD) jedes experimentell ermittelten Wertes wird bei 405 nm gegen den Melanin-Standard gemessen (Standardkurve 0.39 bis 100 µg/ml Melanin, Sigma M8631). Die Meßergebnisse werden angegeben in µg/ml Melanin und in Prozent der negativen unbehandelten Kontrolle.
* NHEM-2 = normal human epidermal melanocytes (Zelltyp)

**Tabelle 1: Ergebnisse des Melanogenese-Assay**

| Verbindungen | Konzentration | Melanin µg/ml | **% Kontrolle** |
|---|---|---|---|
| IBMX¹⁾ | 200µM | 15.39 | **166** |
| Kojicsäure | 560µM | 5.55 | **55** |
| 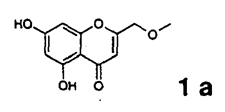 | 450µM | 19.98 | **215** |
| | 150µM | 22.14 | **239** |
| | 50µM | 17.87 | **192** |
| 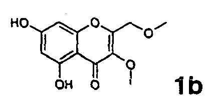 | 200µM | 25.2 | **128** |
| | 40µM | 23.7 | **120** |
| | 8µM | 20.9 | **106** |
| 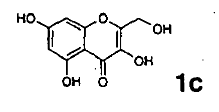 | 200µM | 27.6 | **139** |
| | 40µM | 23.8 | **120** |
| | 8µM | 20.8 | **105** |
| 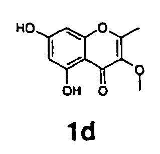 | 40µM | 27.0 | **137** |
| | 8µM | 22.0 | **112** |
| | 1.6µM | 20.8 | **106** |
| 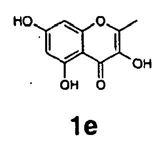 | 200µM | 27.2 | **138** |
| | 40µM | 23.9 | **121** |
| | 8µM | 20.6 | **104** |
| 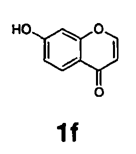 | 40µM | 24.7 | **124** |
| | 8µM | 20.3 | **102** |
| | 1.6µM | 19.9 | **100** |
| 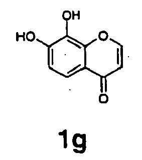 | 8µM | 19.8 | **100** |
| | 1.6µM | 19.9 | **100** |
| | 0.32µM | 20.7 | **104** |

| | | | |
|---|---|---|---|
| *¹⁾IBMX bedeutet 3-Isobutyl-1-methylxanthin* | | | |

### Beispiel 9: Zubereitungen

Im folgenden werden beispielhaft Rezepturen für kosmetische Zubereitungen angegeben, die Verbindungen nach Beispielen 1 - 3 enthalten. Im übrigen sind die INCl-Bezeichnungen der handelsüblichen Verbindungen angegeben.

UV-Pearl , OMC steht für die Zubereitung mit der INCl-Bezeichnung: Water (for EU: Aqua), Ethylhexyl Methoxycinnamate, Silica, PVP, Chlorphenesin, BHT; diese Zubereitung ist im Handel unter der Bezeichnung Eusolex®UV Pearl^{™}OMC von der Merck KGaA, Darmstadt erhältlich.

Die anderen in den Tabellen angegebenen UV-Pearl sind jeweils analog zusammengesetzt, wobei OMC gegen die angegebenen UV-Filter ausgetauscht wurde.

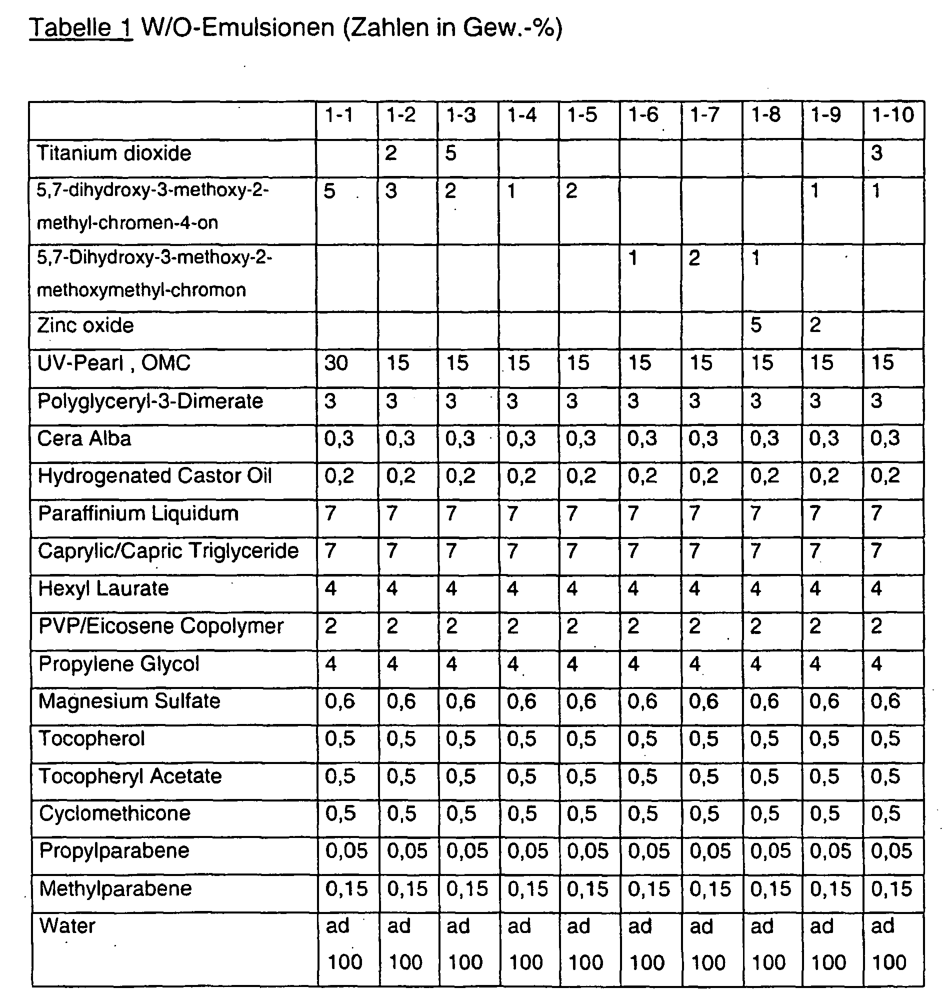

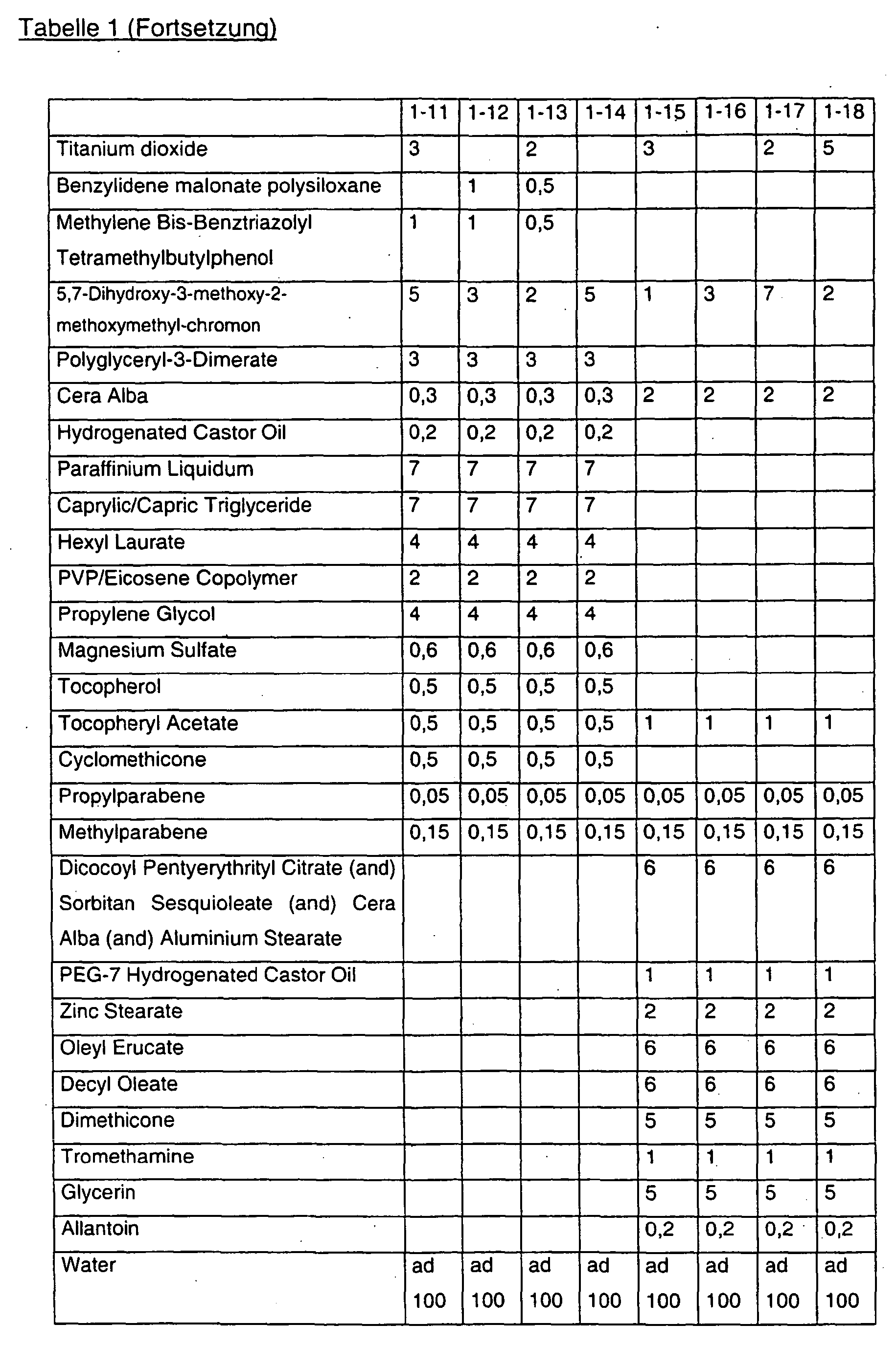

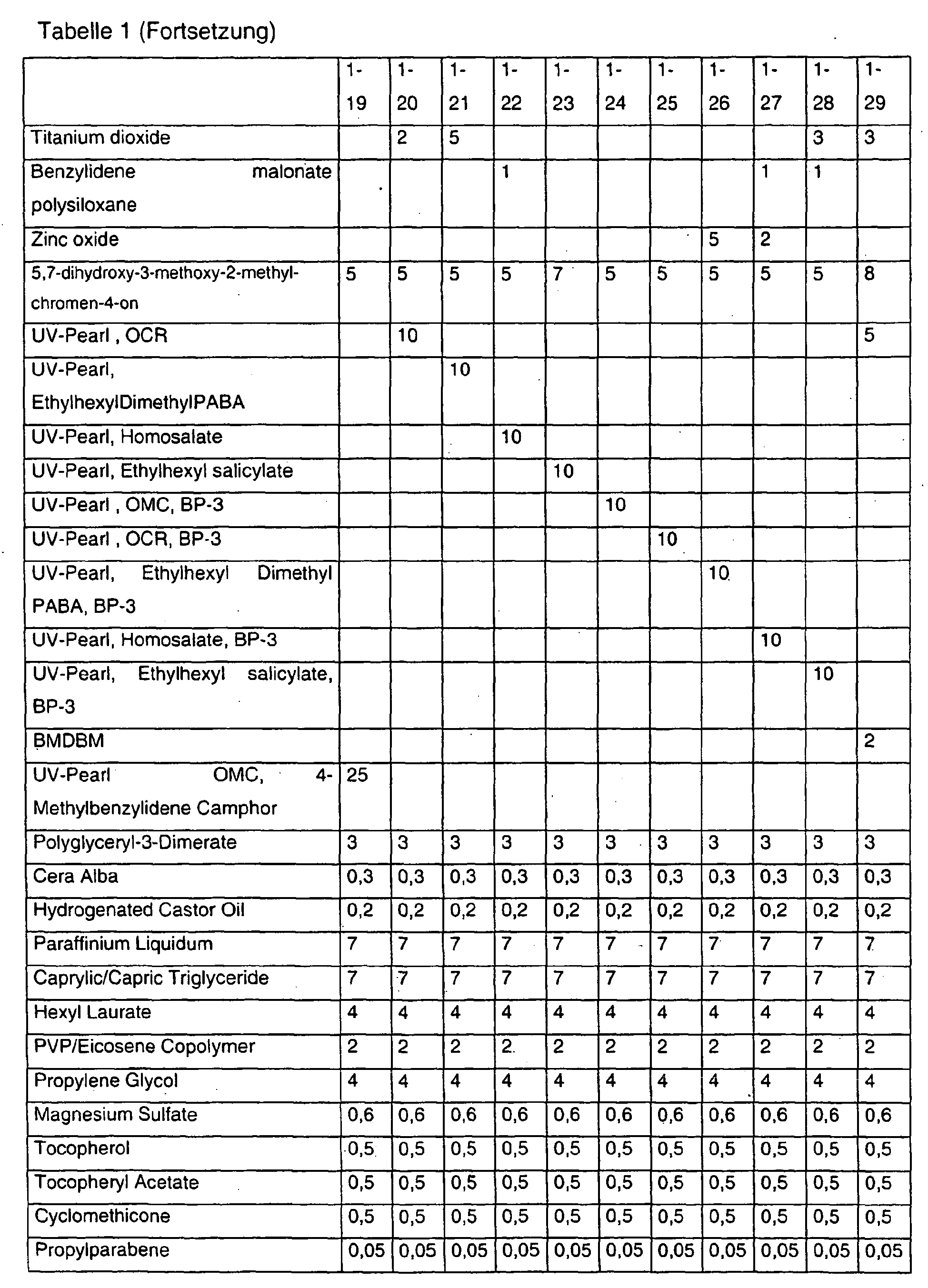

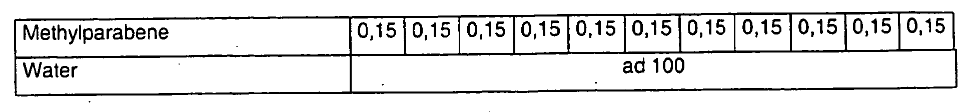

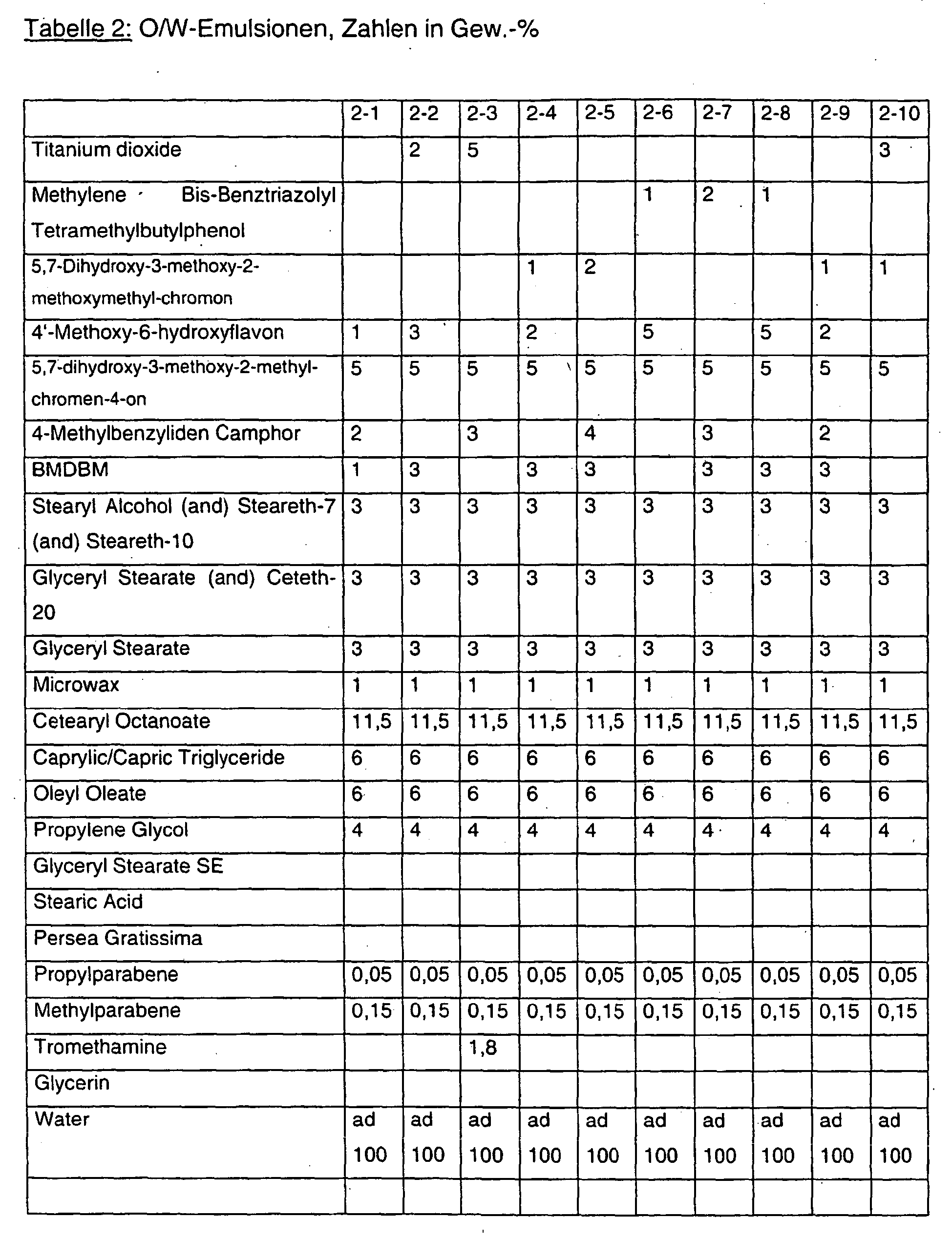

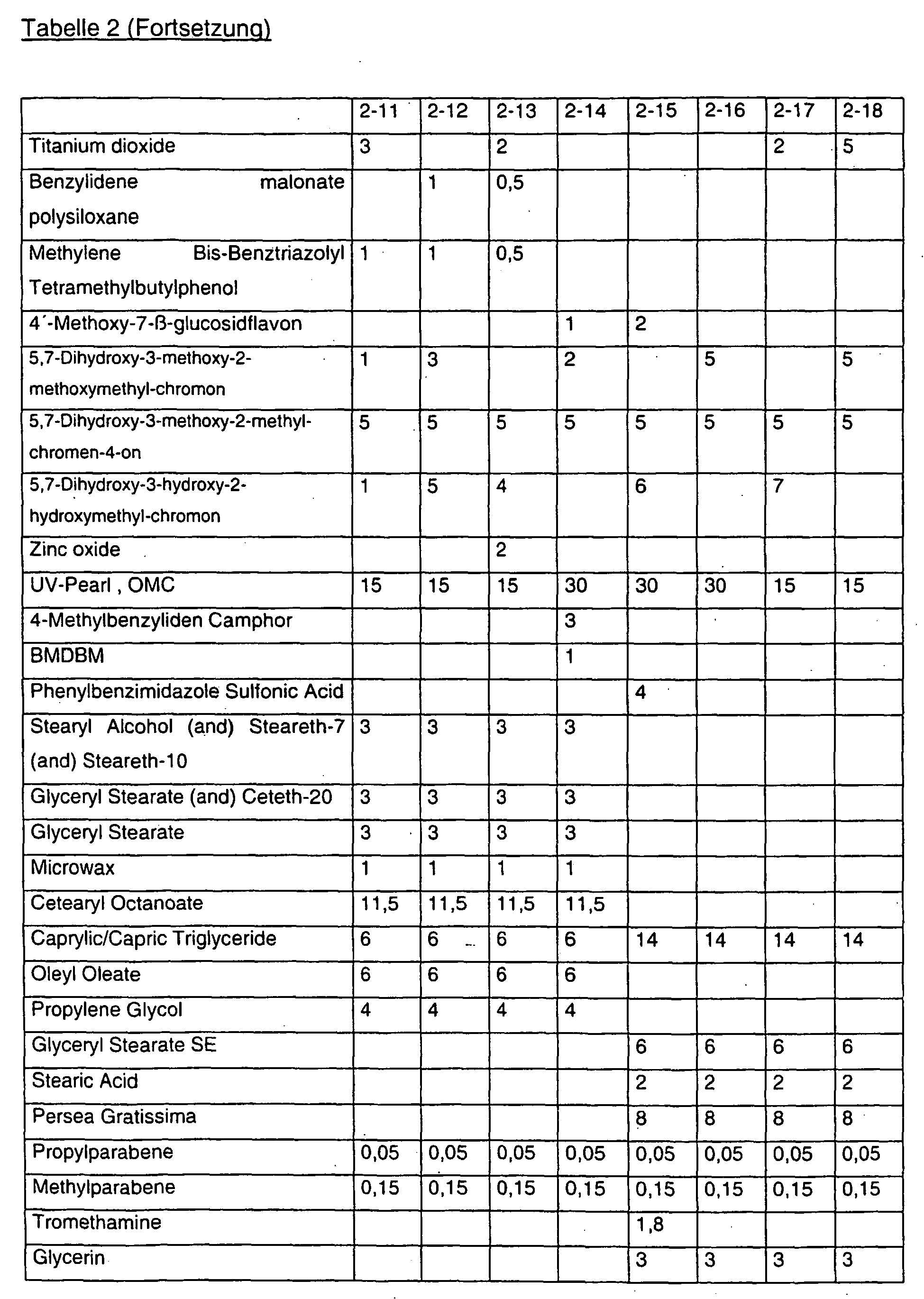

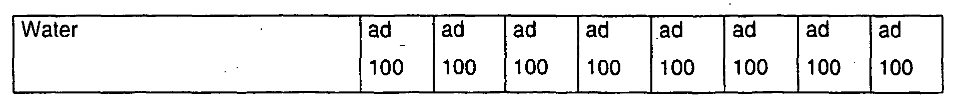

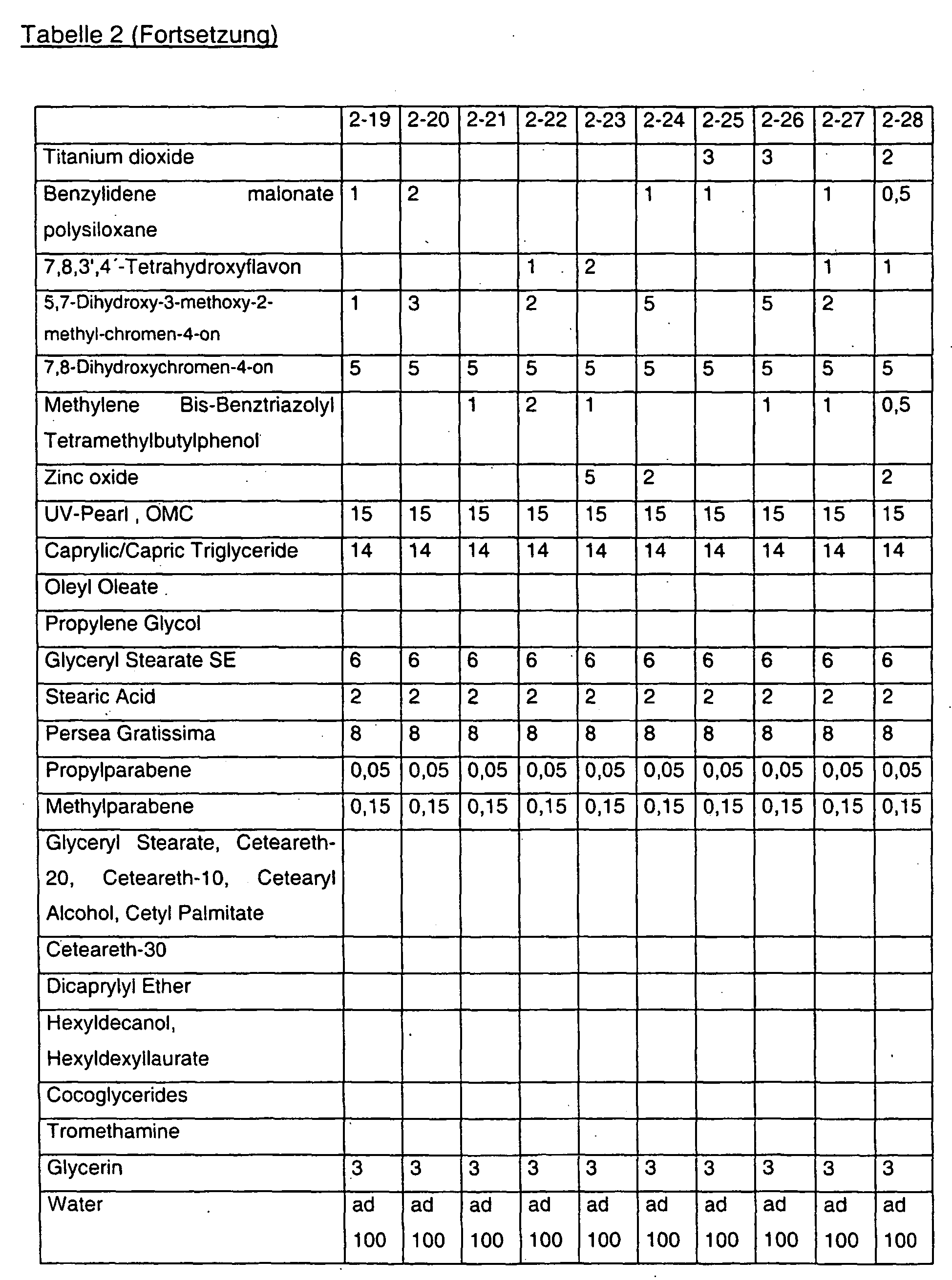

**Tabelle 3: Gele, Zahlen in Gew.-%**

| | 3-1 | 3-2 | 3-3 | 3-4 | 3-5 | 3-6 | 3-7 | 3-8 | 3-9 | 3-10 |
|---|---|---|---|---|---|---|---|---|---|---|
| a = aqueaous gel | | | | | | | | | | |
| Titanium dioxide | | 2 | 5 | | | | | | | 3 |
| 7,8-Dihydroxychromen-4-on | | | | 1 | 2 | | | | 1 | 1 |
| 2-Methyl-3,5,7-trihydroxychromen-4-on | 1 | 3 | | 2 | | 5 | | 5 | 2 | |
| Benzylidene malonate polysiloxane | | | 1 | 1 | 2 | | | | 1 | 1 |
| Methylene Bis-Benztriazolyl Tetramethylbutylphenol | | 1 | | | | 1 | 2 | 1 | | |
| Zinc oxide | | | | 2 | | | | 5 | 2 | |
| UV-Pearl Ethylhexyl Mehtoxycinnamat | 30 | 15 | 15 | 15 | 15 | 15 | 15 | 15 | 15 | 15 |
| 4-Methylbenzyliden Camphor | | | | | 2 | | | | | |
| Butylmethoxydibenzoylmethane | | 1 | | | | | | | | |
| Phenylbenzimidazole Sulfonic Acid | | | 4 | | | | | | | |
| Prunus Dulcis | 5 | 5 | 5 | 5 | 5 | 5 | 5 | 5 | 5 | 5 |
| Tocopheryl Acetate | 0,5 | 0,5 | 0,5 | 0,5 | 0,5 | 0,5 | 0,5 | 0,5 | 0,5 | 0,5 |
| Caprylic/Capric Triglyceride | 3 | 3 | 3 | 3 | 3 | 3 | 3 | 3 | 3 | 3 |
| Octyldodecanol | 2 | 2 | 2 | 2 | 2 | 2 | 2 | 2 | 2 | 2 |
| Decyl Oleate | 2 | 2 | 2 | 2 | 2 | 2 | 2 | 2 | 2 | 2 |
| PEG-8 (and) Tocopherol (and) Ascorbyl Palmitate (and) Ascorbic Acid (and) Citric Acid | 0,05 | 0,05 | 0,05 | 0,05 | 0,05 | 0,05 | 0,05 | 0,05 | 0,05 | 0,05 |
| Sorbitol | 4 | 4 | 4 | 4 | 4 | 4 | 4 | 4 | 4 | 4 |
| Polyacrylamide (and) C13-14 Isoparaffin (and) Laureth-7 | 3 | 3 | 3 | 3 | 3 | 3 | 3 | 3 | 3 | 3 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

### Beispiel 10: Selbstbräunungscreme mit DHA (O/W)

Die Herstellung der Selbstbräunungscreme erfolgt indem man Phase A (bestehend aus Glycerylstearat, Stearylalkohol, Ceterarylalkohol, Cetearylethylhexanoat, Capryltriglycerid, Stearoxydimethicon, Dimethicon, Tocopherylacetat, Propylparaben) und Phase B (bestehend aus Propylenglykol, Methylparaben und Wasser) erhitzt. Phase B wird langsam in Phase A eingerührt und homogenisiert. Unter Rühren wird abgekühlt. Das 5,7-Dihydroxy-3-methoxy-2-methyl-chromen-4-on wird vor dem DHA im Wasser gelöst. Bei 40 °C wird Phase C (bestehend aus DHA, 5,7-Dihydroxy-3-methoxy-2-methyl-chromen-4-on und Wasser) zugegeben.

| **INCI** | **[%]** |
|---|---|
| Aqua (Water) | 69.50 |
| Dihydroxyacetone | 2.00 |
| 5,7-Dihydroxy-3-methoxy-2-methyl-chromen-4-on | 0.50 |
| Methylparaben | 0.15 |
| Propylene glycol | 3.00 |
| Glyceryl stearate, Stearyl alcohol CETEH-20, STEARETH-25 | 8.00 |
| Cetearyl alcohol | 1.50 |
| Cetearyl ethylhexanoate | 6.50 |
| Caprylic/Capric triglyceride | 6.50 |
| Stearoxy dimethicone | 1.20 |
| Dimethicone | 0.50 |
| Tocopheryl acetate | 0.50 |
| Propylparaben | 0.05 |
| Parfum | 0.10 |

## Patentansprüche

1. Verwendung von mindestens einer Verbindung der Formel I 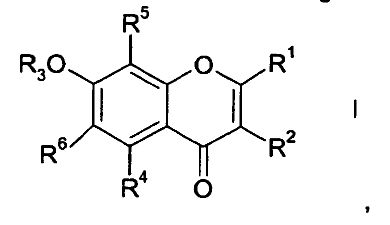 wobei
R¹ und R² gleich oder verschieden sein können und für H, OH, OCH₃, CH₃, CH₂OH oder CH₂OCH₃ stehen,
R³ steht für H oder geradkettige oder verzweigte C₁- bis C₂₀-Alkylgruppen,
R⁴ steht für H oder OR⁸,
R⁵ und R⁶ gleich oder verschieden sein können und ausgewählt sind aus
- -H, -OH,
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und
R⁸ steht für H oder geradkettige oder verzweigte C₁- bis C₂₀-Alkylgruppen,
mit der Maßgabe, dass R² nicht H bedeutet, wenn R¹ CH₃ bedeutet als Selbstbräunungssubstanz.

2. Verwendung nach Anspruch 1, **dadurch gekennzeichnet, dass** in Formel I R¹ für CH₂OCH₃, CH₂OH oder CH₃ steht.

3. Verwendung nach Anspruch 1, **dadurch gekennzeichnet, dass** R² für H, OH oder OCH₃ steht.

4. Verwendung nach einem oder mehreren der Ansprüche 1 bis 3, **dadurch gekennzeichnet, dass** R³ steht für H und R⁴ steht für H oder OH.

5. Verwendung nach einem oder mehreren der Ansprüche 1 bis 4, **dadurch gekennzeichnet, dass** R⁵ für H oder OH und R⁶ für H steht.

6. Verwendung nach einem oder mehreren der Ansprüche 1 bis 5, **dadurch gekennzeichnet, dass** es sich bei der Verbindung nach Formel I um eine Verbindung ausgewählt aus den Verbindungen mit den Formeln la bis Ig handelt:

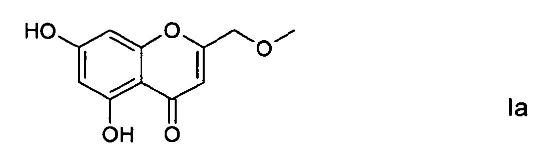

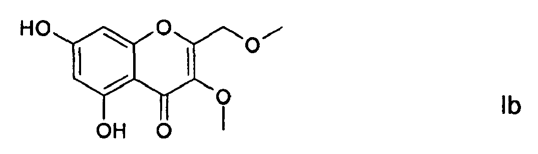

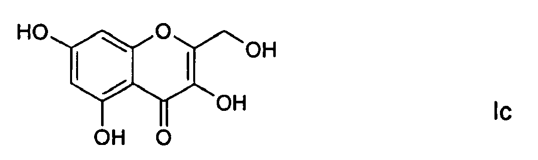

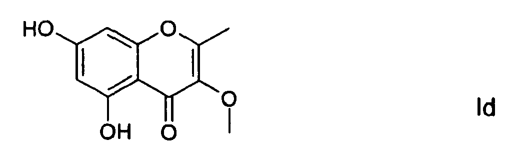

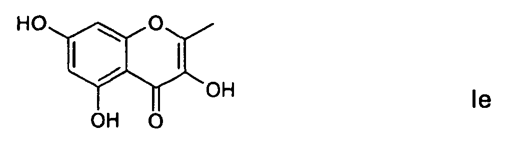

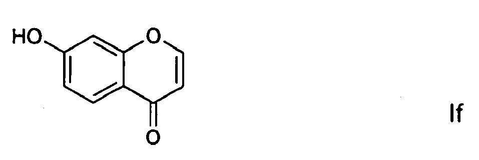

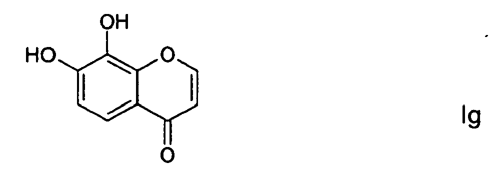

7. Verwendung der Verbindungen der Formel I nach einem oder mehreren der Ansprüche 1 bis 6 zur Steigerung der Melaninsynthese in der Haut.

8. Verbindungen nach Formel I 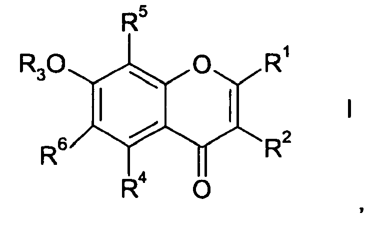 wobei
R¹ CH₂OH oder CH₂OCH₃,
R² OH oder OCH₃ bedeuten,
R³ steht für H oder geradkettige oder verzweigte C₁- bis C₂₀-Alkylgruppen,
R⁴ steht für H oder OR⁸,
R⁵ und R⁶ gleich oder verschieden sein können und ausgewählt sind aus
- H, -OH,
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und
R⁸ steht für H oder geradkettige oder verzweigte C₁- bis C₂₀-Alkylgruppen.

9. Verbindungen nach Anspruch 8, **dadurch gekennzeichnet, dass** R³ für H steht.

10. Verbindungen nach Anspruch 8 oder 9, **dadurch gekennzeichnet, dass** R⁴ für H oder OH steht.

11. Verbindungen nach einem oder mehreren der Ansprüche 8 bis 10, **dadurch gekennzeichnet, dass** R⁵ für H oder OH und R⁶ für H steht.

12. Verbindungen nach einem oder mehreren der Ansprüche 8 bis 11, ausgewählt aus der Verbindung der Formel Ib oder Ic

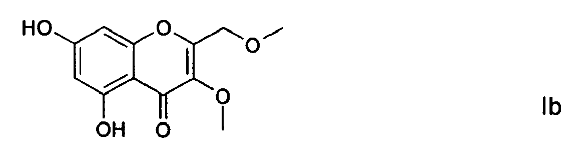

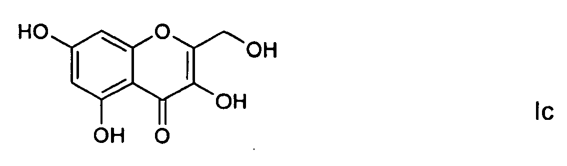

13. Kosmetische und/oder dermatologische Zubereitung enthaltend mindestens eine Verbindung der Formel I nach einem oder mehreren der Ansprüche 8 bis 12 und mindestens einen für topische Anwendungen geeigneten Träger.

14. Selbstbräunungszubereitung enthaltend mindestens eine Verbindung der Formel I nach einem oder mehreren der Ansprüche 1 bis 12 und mindestens einen für topische Anwendungen geeigneten Träger.

15. Zubereitung nach Anspruch 13 oder 14, **dadurch gekennzeichnet, dass** sie eine oder mehrere Verbindungen der Formel I in einer Menge von 0,01 bis 10 Gew.-% enthält.

16. Zubereitung nach einem oder mehreren der Ansprüche 13 bis 15, **dadurch gekennzeichnet, dass** mindestens eine weitere Selbstbräunungssubstanz enthalten ist.

17. Zubereitung nach Anspruch 16, **dadurch gekennzeichnet, dass** die Selbstbräunungssubstanz ausgewählt wird aus der Gruppe Glycerolaldehyd, Hydroxymethylglyoxal, γ-Dialdehyd, Erythrulose, 6-Aldo-D-Fructose, Ninhydrin, 5-Hydroxy-1,4-naphtochinon, 2-Hydroxy-1,4-naphtochinon, 1,3-Diyhydroxyaceton, Dihydroxyacetonphosphat, Glyceraldehydphosphat oder Erythrose.

18. Zubereitung nach einem oder mehreren der Ansprüche 13 bis 17, **dadurch gekennzeichnet, dass** ein oder mehrere Antioxidantien enthalten sind.

19. Zubereitung nach einem oder mehreren der Ansprüche 13 bis 18, **dadurch gekennzeichnet, dass** ein oder mehrere Vitamine enthalten sind.

20. Zubereitung nach einem oder mehreren der Ansprüche 13 bis 19, **dadurch gekennzeichnet, dass** ein oder mehrere UV-Filter enthalten sind.

21. Verfahren zur Herstellung einer Zubereitung nach einem oder mehreren der Ansprüche 13 bis 20, **dadurch gekennzeichnet, dass**
mindestens eine Verbindung der Formel I mit einem für topische Anwendungen geeignetem Träger vermischt wird.

22. Verfahren zur Herstellung einer Verbindung der Formel I nach einem oder mehreren der Ansprüche 8 bis 12, **dadurch gekennzeichnet, dass** die Herstellung durch die Cyclisierung eines o-Hydroxyacetophenones mit einem geeigneten Anhydrid oder mit einem geeignetem Acylchlorid erfolgt.

## Claims

1. Use of at least one compound of the formula I 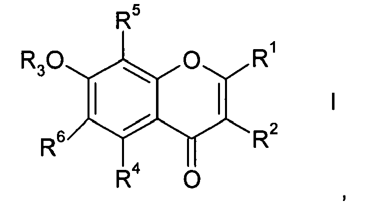 where
R¹ and R² may be identical or different and stand for H, OH, OCH₃, CH₃, CH₂OH or CH₂OCH₃,
R³ stands for H or straight-chain or branched C₁- to C₂₀-alkyl groups, R⁴ stands for H or OR⁸,
R⁵ and R⁶ may be identical or different and are selected from
- -H, -OH,
- straight-chain or branched C₁- to C₂₀-alkyl groups,
- straight-chain or branched C₃- to C₂₀-alkenyl groups,
- straight-chain or branched C₁- to C₂₀-hydroxyalkyl groups, where the hydroxyl group may be bonded to a primary or secondary carbon atom of the chain, and furthermore the alkyl chain may also be interrupted by oxygen, and
R⁸ stands for H or straight-chain or branched C₁- to C₂₀-alkyl groups, with the proviso that R² does not denote H if R¹ denotes CH₃,
as self-tanning substance.

2. Use according to Claim 1, **characterised in that** R¹ in formula I stands for CH₂OCH₃, CH₂OH or CH₃.

3. Use according to Claim 1, **characterised in that** R² stands for H, OH or OCH₃.

4. Use according to one or more of Claims 1 to 3, **characterised in that** R³ stands for H and R⁴ stands for H or OH.

5. Use according to one or more of Claims 1 to 4, **characterised in that** R⁵ stands for H or OH and R⁶ stands for H.

6. Use according to one or more of Claims 1 to 5, **characterised in that** the compound of the formula I is a compound selected from the compounds having the formulae Ia to Ig:

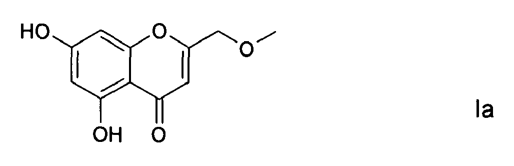

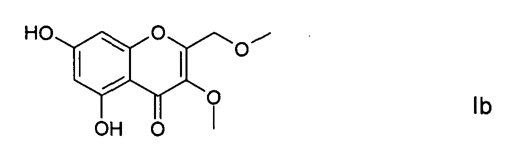

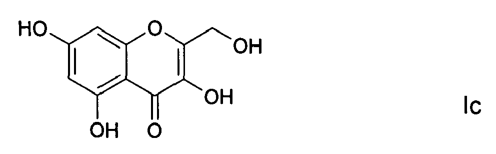

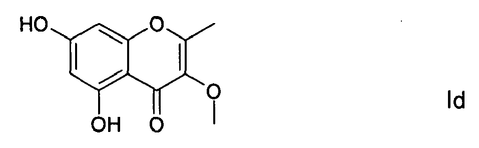

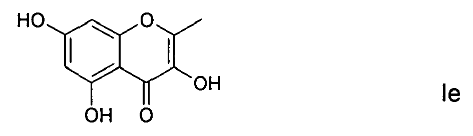

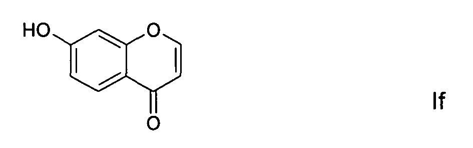

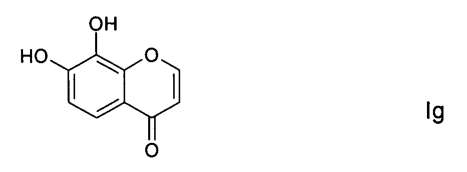

7. Use of the compounds of the formula I according to one or more of Claims 1 to 6 for increasing melanin synthesis in the skin.

8. Compounds of the formula I 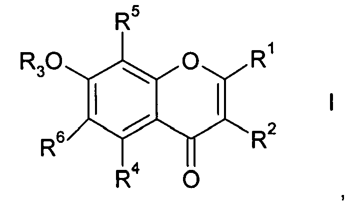 where
R¹ denotes CH₂OH or CH₂OCH₃,
R² denotes OH or OCH₃,
R³ stands for H or straight-chain or branched C₁- to C₂₀-alkyl groups, R⁴ stands for H or OR⁸,
R⁵ and R⁶ may be identical or different and are selected from
- -H, -OH,
- straight-chain or branched C₁- to C₂₀-alkyl groups,
- straight-chain or branched C₃- to C₂₀-alkenyl groups,
- straight-chain or branched C₁- to C₂₀-hydroxyalkyl groups, where the hydroxyl group may be bonded to a primary or secondary carbon atom of the chain, and furthermore the alkyl chain may also be interrupted by oxygen, and
R⁸ stands for H or straight-chain or branched C₁- to C₂₀-alkyl groups.

9. Compounds according to Claim 8, **characterised in that** R³ stands for H.

10. Compounds according to Claim 8 or 9, **characterised in that** R⁴ stands for H or OH.

11. Compounds according to one or more of Claims 8 to 10, **characterised in that** R⁵ stands for H or OH and R⁶ stands for H.

12. Compounds according to one or more of Claims 8 to 11, selected from the compounds of the formulae Ib or Ic

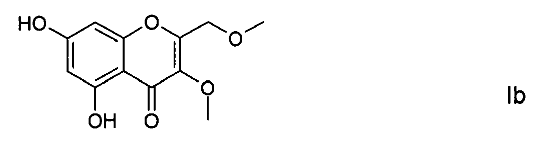

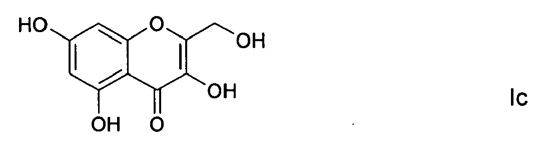

13. Cosmetic and/or dermatological composition comprising at least one compound of the formula I according to one or more of Claims 8 to 12 and at least one vehicle which is suitable for topical applications.

14. Self-tanning composition comprising at least one compound of the formula I according to one or more of Claims 1 to 12 and at least one vehicle which is suitable for topical applications.

15. Composition according to Claim 13 or 14, **characterised in that** it comprises one or more compounds of the formula I in an amount of 0.01 to 10% by weight.

16. Composition according to one or more of Claims 13 to 15, **characterised in that** at least one further self-tanning substance is present.

17. Composition according to Claim 16, **characterised in that** the self-tanning substance is selected from the group glycerolaldehyde, hydroxymethylglyoxal, γ-dialdehyde, erythrulose, 6-aldo-D-fructose, ninhydrin, 5-hydroxy-1,4-naphthoquinone, 2-hydroxy-1,4-naphthoquinone, 1,3-dihydroxyacetone, dihydroxyacetone phosphate, glyceraldehyde phosphate and erythrose.

18. Composition according to one or more of Claims 13 to 17, **characterised in that** one or more antioxidants are present.

19. Composition according to one or more of Claims 13 to 18, **characterised in that** one or more vitamins are present.

20. Composition according to one or more of Claims 13 to 19, **characterised in that** one or more UV filters are present.

21. Process for the preparation of a composition according to one or more of Claims 13 to 20, **characterised in that** at least one compound of the formula I is mixed with a vehicle which is suitable for topical applications.

22. Process for the preparation of a compound of the formula I according to one or more of Claims 8 to 12, **characterised in that** the preparation is carried out by cyclisation of an o-hydroxyacetophenone with a suitable anhydride or with a suitable acyl chloride.

## Revendications

1. Utilisation d'au moins un composé de formule I 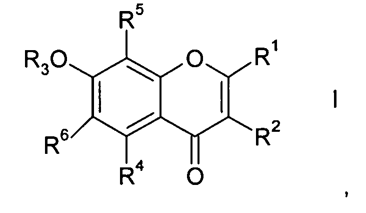 dans laquelle
R¹ et R² peuvent être identiques ou différents et représentent H, OH, OCH₃, CH₃, CH₂OH ou CH₂OCH₃,
R³ représente H ou des groupements C₁- à C₂₀-alkyle à chaîne linéaire ou ramifiée,
R⁴ représente H ou OR⁸,
R⁵ et R⁶ peuvent être identiques ou différents et sont choisis parmi
- -H, -OH,
- des groupements C₁- à C₂₀-alkyle à chaîne linéaire ou ramifiée,
- des groupements C₃- à C₂₀-alcényle à chaîne linéaire ou ramifiée,
- des groupements C₁- à C₂₀-hydroxyalkyle à chaîne linéaire ou ramifiée, où le groupement hydroxyle peut être lié à un atome de carbone primaire ou secondaire de la chaîne, et en outre la chaîne alkyle peut aussi être interrompue par oxygène, et
R⁸ représente H ou des groupements C₁- à C₂₀-alkyle à chaîne linéaire ou ramifiée,
à condition que R² ne désigne pas H si R¹ désigne CH₃,
comme substance auto-bronzante.

2. Utilisation selon la revendication 1, **caractérisée en ce que** R¹ dans la formule I représente CH₂OCH₃, CH₂OH ou CH₃.

3. Utilisation selon la revendication 1, **caractérisée en ce que** R² représente H, OH ou OCH₃.

4. Utilisation selon l'une ou plusieurs des revendications 1 à 3, **caractérisée en ce que** R³ représente H et R⁴ représente H ou OH.

5. Utilisation selon l'une ou plusieurs des revendications 1 à 4, **caractérisée en ce que** R⁵ représente H ou OH et R⁶ représente H.

6. Utilisation selon l'une ou plusieurs des revendications 1 à 5, **caractérisée en ce que** le composé de formule I est un composé choisi parmi les composés répondant aux formules Ia à Ig:

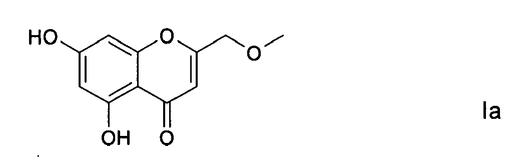

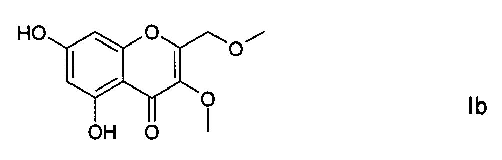

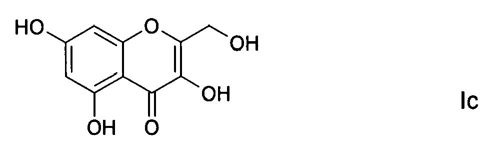

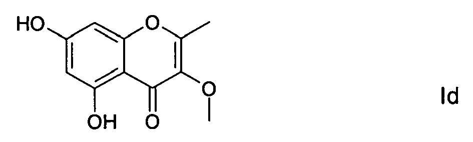

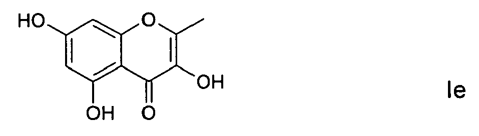

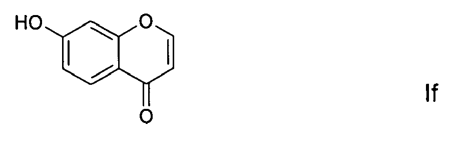

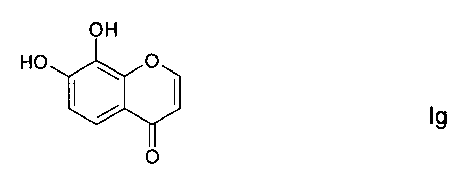

7. Utilisation des composés de formule I selon l'une ou plusieurs des revendications 1 à 6, pour augmenter la synthèse de mélanine dans la peau.

8. Composés de formule I 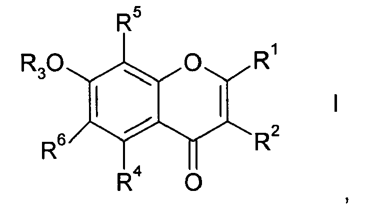 dans laquelle
R¹ désigne CH₂OH ou CH₂OCH₃,
R² désigne OH ou OCH₃,
R³ représente H ou des groupements C₁- à C₂₀-alkyle à chaîne linéaire ou ramifiée,
R⁴ représente H ou OR⁸
R⁵ et R⁶ peuvent être identiques ou différents et sont choisis parmi
- -H, -OH,
- des groupements C₁- à C₂₀-alkyle à chaîne linéaire ou ramifiée,
- des groupements C₃- à C₂₀-alcényle à chaîne linéaire ou ramifiée,
- des groupements C₁- à C₂₀-hydroxyalkyle à chaîne linéaire ou ramifiée, où le groupement hydroxyle peut être lié à un atome de carbone primaire ou secondaire de la chaîne, et en outre la chaîne alkyle peut aussi être interrompue par oxygène, et
R⁸ représente H ou des groupements C₁- à C₂₀-akyle à chaîne linéaire ou ramifiée.

9. Composés selon la revendication 8, **caractérisés en ce que** R³ représente H.

10. Composés selon la revendication 8 ou 9, **caractérisés en ce que** R⁴ représente H ou OH.

11. Composés selon l'une ou plusieurs des revendications 8 à 10, **caractérisés en ce que** R⁵ représente H ou OH et R⁶ représente H.

12. Composés selon l'une ou plusieurs des revendications 8 à 11, choisis parmi les composés de formules Ib ou Ic

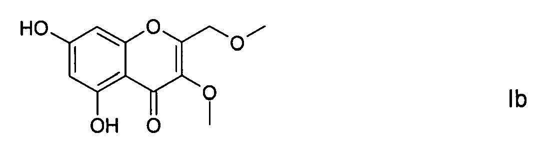

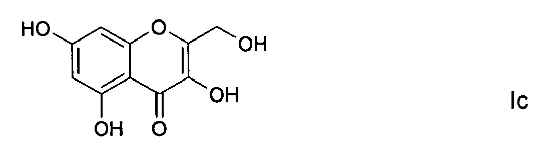

13. Composition cosmétique et/ou dermatologique comprenant au moins un composé de formule I selon l'une ou plusieurs des revendications 8 à 12 et au moins un véhicule qui est convenable pour des applications topiques.

14. Composition auto-bronzante comprenant au moins un composé de formule I selon l'une ou plusieurs des revendications 1 à 12 et au moins un véhicule qui est convenable pour des applications topiques.

15. Composition selon la revendication 13 ou 14, **caractérisée en ce qu'**elle comprend un ou plusieurs composés de formule I selon une quantité allant de 0,01 à 10% en poids.

16. Composition selon l'une ou plusieurs des revendications 13 à 15, **caractérisée en ce qu'**au moins une autre substance auto-bronzante est présente.

17. Composition selon la revendication 16, **caractérisée en ce que** la substance auto-bronzante est choisie dans le groupe constitué par le glycérolaldéhyde, l'hydroxyméthylglyoxal, le γ-dialdéhyde, l'érythrulose, le 6-aldo-D-fructose, la ninhydrine, la 5-hydroxy-1,4-naphtoquinone, la 2-hydroxy-1,4-naphtoquinone, la 1,3-dihydroxyacétone, la dihydroxyacétone phosphate, le glycéraldéhyde phosphate et l'érythrose.

18. Composition selon l'une ou plusieurs des revendications 13 à 17, **caractérisée en ce qu'**un ou plusieurs antioxydants sont présents.

19. Composition selon l'une ou plusieurs des revendications 13 à 18, **caractérisée en ce qu'**une ou plusieurs vitamines sont présentes.

20. Composition selon l'une ou plusieurs des revendications 13 à 19, **caractérisée en ce qu'**un ou plusieurs filtres UV sont présents.

21. Procédé de préparation d'une composition selon l'une ou plusieurs des revendications 13 à 20, **caractérisé en ce qu'**au moins un composé de formule I est mélangé avec un véhicule qui est convenable pour des applications topiques.

22. Procédé de préparation d'un composé de formule I selon l'une ou plusieurs des revendications 8 à 12, **caractérisé en ce que** la préparation est obtenue par cyclisation d'une o-hydroxyacétophénone avec un anhydride convenable ou avec un chlorure d'acyle convenable.
